# EUROPEAN PATENT SPECIFICATION

(11) **EP 0 705 259 B1**
(45) Date of publication and mention of the grant of the patent: **25.02.1998**
(21) Application number: 94920965.4
(22) Date of filing: 20.06.1994
(51) Int. Cl.: C07D 403/06, C07D 401/06, C07D 207/335, C07D 405/14, C07D 401/14, A61K 31/40, A61K 31/445, A61K 31/55

(54) **PHENYRROLE DERIVATIVES AND THEIR USE AS DOPAMINE D3 ANTAGONISTS**
PHENYLPYRROL-DERIVATE UND IHRE VERWENDUNG ALS DOPAMIN D3 ANTAGONISTEN
DERIVES DE PHENYRROLE ET LEUR UTILISATION COMME ANTAGONISTES DE DOPAMINE D3

(30) Priority: 25.06.1993 GB 9313185; 04.02.1994 GB 9402196
(43) Date of publication of application: 10.04.1996
(73) Proprietor: SMITHKLINE BEECHAM PLC, Brentford, Middlesex TW8 9EP (GB)
(72) Inventor: STEMP, Geoffrey, SmithKline Beecham Pharmaceut., Harlow, Essex CM19 5AD (GB); JOHNSON, Christopher Norbert, SmithKline Beecham, Harlow, Essex CM19 5AD (GB); MARKWELL, Roger Edward, SmithKline Beecham Pharm., Harlow, Essex CM19 5AD (GB); WADSWORTH, Harry John, SmithKline Beecham Pharm., Harlow, Essex CM19e5AD (GB); WATTS, Eric Alfred, SmithKline Beecham Pharmaceut., Harlow, Essex CM19 5AD (GB)
(74) Representative: Florence, Julia Anne
(86) International application number: EP9402027
(87) International publication number: WO9500508

(56) References cited:
- EP-A- 0 241 053
- EP-A- 0 259 930
- WO-A-94/03426
- JOURNAL OF MEDICINAL CHEMISTRY, vol.31, no.10, October 1988, WASHINGTON US pages 1934 - 1940 I. VAN WIJNGAARDEN ET AL. '2-Phenylpyrroles as conformationally restricted benzamide analogues. A new class of potential antipsychotics. 2'

## Description

The present invention relates to novel phenylpyrrole derivatives, processes for their preparation, pharmaceutical compositions containing them and their use in therapy, in particular as antipsychotic agents.

European Patent Application No. 241053, describes compounds of the formula : 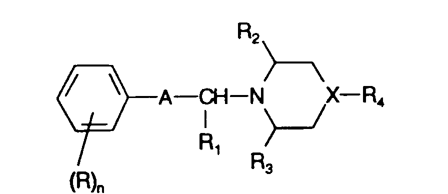 wherein A is an unsaturated 5-membered heterocyclic ring, such as 2,5-pyrrolyl, or 3,5- or 1,4- pyrazolyl; X is a nitrogen or carbon atom; R₁,R₂, R₃ are each hydrogen or alkyl; R₄ is aryl, heteroaryl, arylcarbonyl or heteroaryl-carbonyl; R is selected from a variety of substituents and n is 0-4. The compounds are said to have antipsychotic properties.

European Patent Application No. 259930 describes compounds of the formula : 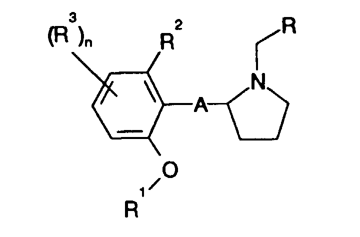 wherein A is an unsaturated 5-membered heterocyclic ring, such as 2,5-pyrrolyl, 1,4-pyrazolyl or 2,5-furyl; R is hydrogen, alkyl or optionally substituted phenyl; R¹ is alkyl, alkenyl or forms a ring with the phenyl group; R² is hydrogen, hydroxy or alkoxy; R³ is selected from a variety of substituents and n is 0-3. These compounds are also said to have antipsychotic properties.

We have now found a novel class of 2-phenylpyrroles which exhibit dopamine antagonist activity and thus have potential as antipsychotic agents.

In a first aspect the present invention provides compounds of formula (I) : 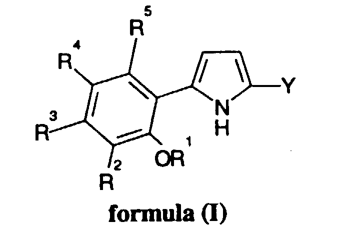 wherein
- R¹: represents C₁₋₄alkyl; and
- R², R³, R⁴ and R⁵: each independently represent hydrogen, halogen, C₁₋₄alkyl, C₁₋₄alkoxy, C₁₋₄alkoxyC₁₋₄alkyl, C₁₋₄alkylsulphonyl, trifluoromethylsulphonyl; optionally substituted arylsulphonyl, optionally substituted heteroarylsulphonyl, optionally substituted aralkylsulphonyl, optionally substituted heteroaralkylsulphonyl, nitro, cyano, amino, mono- or di-alkylamino, trifluoromethyl, trifluoromethoxy, hydroxyl, hydroxyalkyl, C₁₋₄alkylthio, C₁₋₄alkanoyl, C₁₋₄alkoxycarbonyl, or -SO₂NR⁶R⁷ wherein R⁶ and R⁷ each independently represent hydrogen, C₁₋₄alkyl or C₁₋₄alkoxyC₁₋₄alkyl, or NR⁶R⁷ forms a 3- to 8- membered fully saturated heterocyclic ring, a 5- to 8-membered partially saturated heterocyclic ring, or a 5- to 8-membered fully saturated heterocyclic ring which contains in addition to the nitrogen atom an oxygen or sulphur atom; or
- R¹ and R²: together form a linking chain -(CH₂)ₘOₚ;
(wherein m is 2 to 4 and p is zero or 1) which chain may be optionally substituted by one or two C₁₋₄alkyl groups; and
- Y: represents a group of formula : 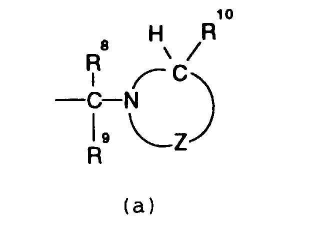 wherein
- R⁸ and R⁹: independently represent hydrogen, C₁₋₆alkyl, optionally substituted arylC₁₋₆alkyl or optionally substituted heteroarylC₁₋₆alkyl;
- R¹⁰: represents an optionally substituted aryl or optionally substituted heteroaryl group; and
- Z: represents -(CH₂)ₙ wherein n is 2 to 8 or -(CH₂)_{q}CHCH(CH₂)ᵣ where q and r independently represent 1 to 3;
and salts thereof.

In the compounds of formula (I) an alkyl group or moiety may be straight or branched. Alkyl groups which may be employed include methyl, ethyl, n-propyl, n-butyl, n-pentyl, n-hexyl and any branched isomers thereof such as isopropyl, t-butyl, sec-pentyl, and the like.

A halogen atom present in the compounds of formula (I) may be a fluorine, chlorine, bromine or iodine atom.

Representative aryl groups or moieties present in any of the substituents R², R³, R⁴, R⁵, R⁸, R⁹, and R¹⁰ in compounds of formula (I) include phenyl, naphthyl, and tetrahydronaphthyl. Suitable examples of heteroaryl groups include both 5 and 6-membered heterocycles containing one or more oxygen, sulphur or nitrogen atoms, such as furyl, thienyl, pyrryl, oxazolyl, thiazolyl, imidazolyl, oxadiazolyl, thiadiazolyl, pyridyl, pyridazyl, pyrimidyl and pyrazyl. Suitable substituents for said aryl and heteroaryl groups include halogen, C₁₋₄alkyl, C₁₋₄alkoxy, C₁₋₄alkoxyC₁₋₄alkyl, nitro, cyano, amino, mono- or dialkylamino, trifluoromethyl, trifluoromethoxy, hydroxy, hydroxyalkyl, C₁₋₄alkanoyl and C₁₋₄alkoxycarbonyl.

The ring 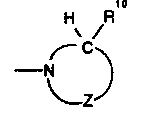 in the group Y preferably has from 4 to 10, e.g. 5 to 8 ring members. Representative heterocyclic groups include pyrrolidinyl, piperidinyl, azacycloheptyl and azacyclooctyl. For the avoidance of doubt the azacycloheptyl and azacyclooctyl rings may also be named as hexahydroazepinyl and octahydroazocinyl respectively.

When R¹ and R² together form a group -(CH₂)ₘOₚ wherein p is 1 it will be appreciated that the oxygen atom is attached to the phenyl ring at the R² position : 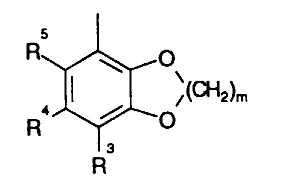 When the (CH₂)ₘ moiety is substituted by two C₁₋₄alkyl groups these are preferably substituted on the same carbon atom e.g. a gem-dimethyl substituent.

In the group -S0₂NR⁶R⁷, when NR⁶R⁷ forms a 3- to 8- membered fully saturated heterocyclic ring this may be for example an azetidinyl, pyrrolidinyl, piperidinyl or azacycloheptyl ring. Examples of a 5- to 8-membered partially saturated heterocyclic ring include 1,2,3,6-tetrahydropyridinyl, and examples of a 5- to 8-membered fully saturated heterocyclic ring which contains in addition to the nitrogen atom an oxygen or sulphur atom include morpholinyl or thiomorpholinyl).

In the compounds of formula (I) R¹ preferably represents methyl or ethyl.

Preferably at least one of R² to R⁵ is hydrogen, and the other substituents are selected from halogen, C₁₋₂alkyl, C₁₋₂alkoxy, C₁₋₄alkylsulphonyl, phenylsulphonyl, benzylsulphonyl and -S0₂NR⁶R⁷ wherein R⁶ and R⁷ represent C₁₋₄alkyl (e.g. methyl, ethyl, n-propyl or iso-propyl) or C₁₋₂alkoxyC₁₋₂alkyl (e.g. methoxyethyl), or NR⁶R⁷ represents a ring selected from azetidinyl, pyrrolidinyl, piperidinyl, azacycloheptyl, 1,2,3,6-tetrahydropyridinyl, 4-morpholinyl or 4-thiomorpholinyl.

In particular one of R² to R⁵, e.g. R⁴, represents C₁₋₄alkylsulphonyl, phenylsulphonyl, benzylsulphonyl or a group -S0₂NR⁶R⁷ and the remaining substituents R² to R⁵ are selected from hydrogen, halogen, C₁₋₂alkyl and C₁₋₂alkoxy. Thus, for example, R² may be hydrogen, halogen e.g. bromine, methyl or methoxy and R³ and R⁵ may be hydrogen.
- R⁸: preferably represents hydrogen.
- R⁹: preferably represents hydrogen or methyl.
- R¹⁰: preferably represents optionally substituted phenyl. Preferred substituents for the group R¹⁰ include halogen (e.g fluorine) C₁₋₄alkyl (e.g. methyl) and C₁₋₄alkoxy (e.g. methoxy).
- Z: preferably represents -(CH₂)ₙ wherein n is 3, 4 or 5, or -(CH₂)_{q}CH=CH(CH₂)ᵣ where the sum of q and r is 2 or 3.

Preferably the compounds of formula (I) are of the R configuration about the carbon atom in the group Y which is substituted by R¹⁰.

It will be appreciated that for use in medicine the salts of formula (I) should be physiologically acceptable. Suitable physiologically acceptable salts will be apparent to those skilled in the art and include for example acid addition salts formed with inorganic acids eg. hydrochloric, hydrobromic, sulphuric, nitric or phosphoric acid; and organic acids eg. succinic, maleic, acetic, fumaric, citric, tartaric, benzoic, p-toluenesulphonic, methanesulphonic or naphthalenesulphonic acid. Other non-physiologically acceptable salts eg. oxalates may be used, for example in the isolation of compounds of formula (I) and are included within the scope of this invention. Also included within the scope of the invention are solvates and hydrates of compounds of formula (I).

It will be appreciated that the compounds of formula (I) contain one or more asymmetric centres and will therefore exist in the form of optical isomers (enantiomers). The present invention includes within its scope all such enantiomers and mixtures, including racemic mixtures, thereof. In addition, all possible diastereomeric forms (individual diastereomers and mixtures thereof) of compounds of formula (I) are included within the scope of the invention.

Particular compounds according to the invention include :
2-(5-ethylsulphonyl-2-methoxyphenyl)-5-(1-(2-(R,S)-phenylpiperidinyl)-methyl)-1H-pyrrole,
2-(5-ethylsulphonyl-2-methoxyphenyl)-5-(1-(2-(R,S)-phenylpyrrolidinyl)-methyl)-1H-pyrrole,
2-(5-ethylsulphonyl-2-methoxyphenyl)-5-(1-(2-(R,S)-phenylazacycloheptyl)-methyl)-1H-pyrrole,
2-(3,5-dibromo-2-methoxyphenyl)-5-(1-(2-(R,S)-phenylazacycloheptyl)-methyl)-1H-pyrrole,
2-(2-methoxy-5-N,N-dimethylaminosulphonylphenyl)-5-[1-(2-(R,S)-phenylazacycloheptyl)methyl]-1H-pyrrole,
2-(3-bromo-5-ethylsulphonyl-2-methoxyphenyl)-5-(1-(2-(R,S)-phenylazacycloheptyl)methyl)-1H-pyrrole,
2-(5-ethylsulphonyl-2-methoxyphenyl)-5-(1-(2-(R)-phenylazacycloheptyl)methyl)-1H-pyrrole
2-(5-ethylsulphonyl-2-methoxyphenyl)-5-(1-(2-(S)-phenylazacycloheptyl)methyl)-1H-pyrrole,
2-(3-bromo-5-ethylsulphonyl-2-methoxyphenyl)-5-(1-(2-(R,S)-phenylazacycloheptyl)methyl)-1H-pyrrole,
2-(3-bromo-5-ethylsulphonyl-2-methoxyphenyl)-5-(1-(2-(R)-phenylazacycloheptyl)methyl)-1H-pyrrole,
2-(3-bromo-5-ethylsulphonyl-2-methoxyphenyl)-5-(1-(2-(S)-phenylazacycloheptyl)methyl)-1H-pyrrole,
2-(2-methoxy-5-dimethylaminosulphonylphenyl)-5-[1-(2-(R,S)-(4-fluorophenyl)azacycloheptyl)methyl]-1H-pyrrole,
2-(3-bromo-5-ethylsulphonyl-2-methoxyphenyl)-5-[1-(2-(R,S)-(4-fluorophenyl)azacycloheptyl)methyl]-1H-pyrrole,
2-(5-ethylsulphonyl-2-methoxyphenyl)-5-[1-(2-(R,S)-(4-methylphenyl)azacycloheptyl)-methyl]-1H-pyrrole),
2-(3-bromo-5-ethylsulphonyl-2-methoxyphenyl)-5-[1-(2-(R,S)-(4-methoxyphenyl)-azacycloheptyl)methyl]-1H-pyrrole,
2-(5-N,N-diethylaminosulphonyl-2-methoxyphenyl)-5-[1-(2-(R,S)-phenylazacycloheptyl)-methyl]-1H-pyrrole,
2-(2-methoxy-5-pyrrolidinylsulphonylphenyl)-5-[1-(2-(R,S)-phenylazacycloheptyl)methyl]-1H-pyrrole,
2-(2-methoxy-5-piperidinylsulphonylphenyl)-5-[1-(2-(R,S)-phenylazacycloheptyl)-methyl]-1H-pyrrole,
2-(2-methoxy-5-(4-morpholinyl)sulphonylphenyl)-5-[1(2-(R,S)-phenylazacycloheptyl)methyl]-1H-pyrrole,
2-(2-methoxy-5-(4-morpholinyl)sulphonylphenyl)-5-(1-(2-(R)-phenylazacycloheptyl)-methyl)-1H-pyrrole,
2-(2-methoxy-5-(4-morpholinyl)suphonylphenyl)-5-(1-(2-(S)-phenylazacycloheptyl)methyl)-1H-pyrrole,
2-(2-methoxy-5-N,N-di-n-propylaminosulphonylphenyl)-5-[1-(2-(R,S)-phenylazacycloheptyl)methyl]-1H-pyrrole,
2-(2-methoxy-5-N,N-di-n-propylaminosuphonylphenyl)-5-(1-(2-(R)-phenylazacycloheptyl)methyl)-1H-pyrrole,
2-(2-methoxy-5-N,N-di-n-propylaminosuphonylphenyl)-5-(1-(2-(S)-phenyl-azacycloheptyl)methyl)-1H-pyrrole,
2-(2-methoxy-5-(4-thiomorpholinyl)sulphonylphenyl)-5-[1-(2-(R,S)-phenylazacycloheptyl)methyl]-1H-pyrrole,
2-(5-ethylsulphonyl-2-methoxyphenyl)-5-(1-(2-(R)-phenylpiperidinyl)methyl)-1H-pyrrole,
2-(5-ethylsulphonyl-2-methoxyphenyl)-5-( 1-(2-(S)-phenylpiperidinyl)methyl)-1H-pyrrole,
(-)-2-(5-ethylsulphonyl-2-methoxyphenyl)-5-(1-(2-(R or S)-phenylpyrrolidinyl)methyl)-1H-pyrrole,
(+)-2-(5-ethylsulphonyl-2-methoxyphenyl)-5-(1-(2-(R or S)-phenylpyrrolidinyl)methyl)-1H-pyrrole,
2-[5-(azetidinylsulphonyl)-2-methoxyphenyl]-5-[1(2-(R,S)-phenylazacycloheptyl)-methyl]-1H-pyrrole,
2-[5-(bis-(2-methoxyethyl))aminosulphony-2-methoxyphenyl]-5-(1-(2-(R,S)-phenylazacycloheptyl)methyl)-1H-pyrrole,
2-{5-[(N-ethyl-N-2-methoxyethyl)aminosulphonyl]-2-methoxyphenyl)}-5-[1-(2-(R,S)-phenylazacycloheptyl)methyl]-1H-pyrrole,
2-(5-benzylsulphonyl-2-methoxyphenyl)-5-[1-(2-(R,S)-phenylazacycloheptyl)methyl]-1H-pyrrole,
2-(5-isopropylsulphonyl-2-methoxyphenyl)-5-[1-(2-(R,S)-phenylazacycloheptyl)methyl]-1H-pyrrole,
2-(2-methoxy-5-phenylsulphonylphenyl)-5-[1-(2-(R,S)-phenylazacycloheptyl)methyl]-1H-pyrrole,
2-[5-(3,6-dihydro-2H-pyridine-1-sulphonyl)-2-methoxyphenyl]-5-[1-(2-(R,S)-phenylazacycloheptyl)methyl]-1H-pyrrole,
and salts thereof.

The present invention also provides a process for preparing compounds of formula (I) which process comprises:
(a) carrying out a Mannich reaction with a compound of formula (II) : 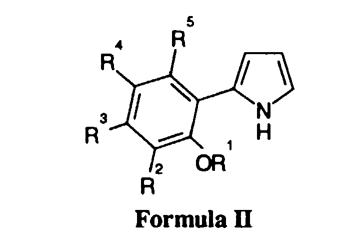 (wherein R¹-R⁵ are as hereinbefore defined),
   and an amine of formula (III): 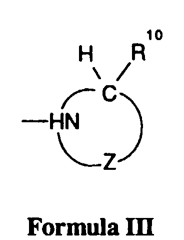 wherein R¹⁰ and Z are as hereinbefore defined;
(b) carrying out a Vilsmeier reaction with a compound of formula (II) and an amide of formula (IV): 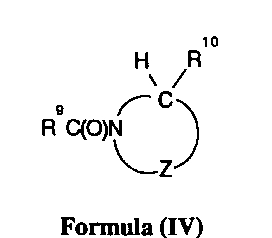 wherein R⁹, R¹⁰ and Z are as hereinbefore defined;
   and reducing the intermediate product with, for example, sodium borohydride or cyanoborohydride;
c) to prepare a compound of formula (I) in which R⁸ and R⁹ both represent hydrogen, reductive amination of a compound of formula (V) : 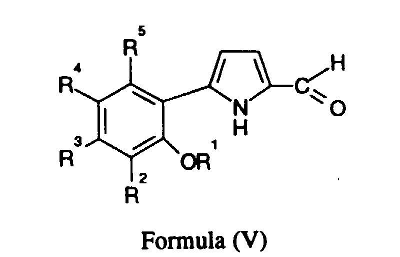 with an amine of formula (III);
   and optionally thereafter forming a salt of formula (I).

The Mannich reaction according to process (a) may be effected according to conventional methods. Thus for example the cyclic amine of formula (III) may first be reacted with formaldehyde and the product subsequently reacted with a compound of formula (II). The reaction is preferably effected in a protic solvent, for example an alcohol such as ethanol. An organic or inorganic acid, e.g. acetic acid may be employed as a catalyst.

The Vilsmeier reaction according to process (b) may also be effected according to conventional methods. Thus, for example, the amide of formula (IV) may first be treated with phosphorus oxychloride (POCl₃) and the resulting product subsequently reacted with a compound of formula (II) conveniently in a solvent such as dichloroethane or dichloromethane. The product of this reaction is then reduced with, for example, sodium borohydride or cyanoborohydride. The reduction may be carried out in a suitable solvent, for example dichloroethane, dichloromethane, methanol, ethanol, water or mixtures thereof.

Reductive amination according to process (c) will generally be carried out using a reducing agent such as sodium borohydride or cyanoborohydride and in the presence of a Lewis acid such as titanium (IV) chloride. Reaction of a compound (V) with the amine may conveniently be effected in a solvent such as dichloromethane or dichloroethane.

A compound of formula (II) may be prepared by cyclisation of a dicarbonyl compound of formula (VI): 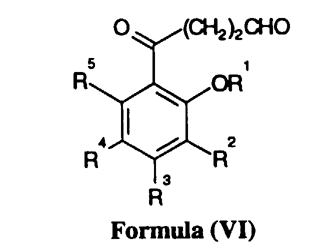 wherein R¹-R⁵ are as hereinbefore defined.

The reaction may be effected using an ammonium salt, e.g. ammonium acetate, in a solvent such as ethanol. (See, for example, C.G. Kruse et al., Heterocycles, vol 26, 3141, 1987).

A compound of formula (VI) may itself be prepared by reacting the appropriate substituted benzoyl halide with a metallo derivative of a 2-(2-haloethyl)-1,3-dioxolane, or 2-(2-haloethyl)-1,3-dioxane, followed by acid hydrolysis.

Compounds of formula (III) are available commercially or may be prepared by standard methods.

A compound of formula (IV) may be prepared by reacting an amine of formula (III) with acetic anhydride in formic acid.

A compound of formula (V) may be prepared by carrying out a Vilsmeier reaction in which dimethylformamide is reacted with phosphorus oxychloride and the product reacted with a compound of formula (II), in a solvent such as dichloroethane, followed by acid hydrolysis.

When a compound of formula (I) is obtained as a mixture of enantiomers these may be separated by conventional methods such as crystallisation in the presence of a resolving agent, or chromatography, for example using a chiral HPLC column.

Alternatively a compound of formula (I) may be prepared as a single enantiomer by employing a chiral amine in the synthesis, for example directly in process (a) or (c) or in the preparation of an amide for use in process (b). A chiral amine of formula (III), (IV) or (V) may be prepared by resolving an enantiomeric mixture of the appropriate amine for example by coupling to a chiral auxiliary such as (S)-(+)-α-methoxyphenylacetic acid and separating the resulting diastereoisomers by chromatography. The auxiliary moiety may be removed to give the desired chiral amine. Thus for example the (S)-(+)-α-methoxyphenylacetyl moiety may be cleaved under basic conditions for example using methyl lithium in a solvent such as tetrahydrofuran or hexane.

Compounds of formula (I) have been found to exhibit affinity for dopamine receptors, in particular D₃ receptors, and are expected to be useful in the treatment of disease states which require modulation of such receptors, such as psychotic conditions. The therapeutic effect of currently available antipsychotic agents (neuroleptics) is generally believed to be exerted via blockade of D₂ receptors; however this mechanism is also thought to be responsible for undesirable extrapyramidal side effects (eps) associated with many neuroleptic agents. Without wishing to be bound by theory, it has been suggested that blockade of the recently characterised dopamine D₃ receptor may give rise to beneficial antipsychotic activity without significant eps. (see for example Sokoloff et al, Nature, 1990; 347: 146-151; and Schwartz et al, Clinical Neuropharmacology, Vol 16, No. 4, 295-314, 1993). Preferred compounds of the present invention are therefore those which have higher affinity for dopamine D₃ than dopamine D₂ receptors (such affinity can be measured using standard methodology for example using cloned dopamine receptors). Said compounds may advantageously be used as selective modulators of D₃ receptors. In particular compounds of formula (I) are dopamine D₃ receptor antagonists and as such are of potential use as antipsychotic agents for example in the treatment of schizophrenia, schizo-affective disorders, psychotic depression and mania. Other conditions which may be treated by modulation of dopamine D₃ receptors include dyskinetic disorders such as Parkinson's disease, neuroleptic-induced parkinsonism and tardive dyskinesias; depression; and drug (eg. cocaine) dependency.

The invention also provides the use of a compound of formula (I) or a physiologically acceptable salt thereof in the manufacture of a medicament for the treatment of conditions which require modulation of dopamine D₃ receptors, for example psychoses such as schizophrenia.

For use in medicine, the compounds of the present invention are usually administered as a standard pharmaceutical composition. The present invention therefore provides in a further aspect pharmaceutical compositions comprising a compound of formula (I) or a physiologically acceptable salt thereof and a physiologically acceptable carrier.

The compounds of formula (I) may be administered by any convenient method, for example by oral, parenteral, buccal, sublingual, nasal, rectal or transdermal administration and the pharmaceutical compositions adapted accordingly.

The compounds of formula (I) and their physiologically acceptable salts which are active when given orally can be formulated as liquids or solids, for example syrups, suspensions or emulsions, tablets, capsules and lozenges.

A liquid formulation will generally consist of a suspension or solution of the compound or physiologically acceptable salt in a suitable liquid carrier(s) for example an aqueous solvent such as water, ethanol or glycerine, or a non-aqueous solvent, such as polyethylene glycol or an oil. The formulation may also contain a suspending agent, preservative, flavouring or colouring agent.

A composition in the form of a tablet can be prepared using any suitable pharmaceutical carrier(s) routinely used for preparing solid formulations. Examples of such carriers include magnesium stearate, starch, lactose, sucrose and cellulose.

A composition in the form of a capsule can be prepared using routine encapsulation procedures. For example, pellets containing the active ingredient can be prepared using standard carriers and then filled into a hard gelatin capsule; alternatively, a dispersion or suspension can be prepared using any suitable pharmaceutical carrier(s), for example aqueous gums, celluloses, silicates or oils and the dispersion or suspension then filled into a soft gelatin capsule.

Typical parenteral compositions consist of a solution or suspension of the compound or physiologically acceptable salt in a sterile aqueous carrier or parenterally acceptable oil, for example polyethylene glycol, polyvinyl pyrrolidone, lecithin, arachis oil or sesame oil. Alternatively, the solution can be lyophilised and then reconstituted with a suitable solvent just prior to administration.

Compositions for nasal administration may conveniently be formulated as aerosols, drops, gels and powders. Aerosol formulations typically comprise a solution or fine suspension of the active substance in a physiologically acceptable aqueous or non-aqueous solvent and are usually presented in single or multidose quantities in sterile form in a sealed container, which can take the form of a cartridge or refill for use with an atomising device. Alternatively the sealed container may be a unitary dispensing device such as a single dose nasal inhaler or an aerosol dispenser fitted with a metering valve which is intended for disposal once the contents of the container have been exhausted. Where the dosage form comprises an aerosol dispenser, it will contain a propellant which can be a compressed gas such as compressed air or an organic propellant such as a fluorochlorohydrocarbon. The aerosol dosage forms can also take the form of a pump-atomiser.

Compositions suitable for buccal or sublingual administration include tablets, lozenges and pastilles, wherein the active ingredient is formulated with a carrier such as sugar and acacia, tragacanth, or gelatin and glycerin.

Compositions for rectal administration are conveniently in the form of suppositories containing a conventional suppository base such as cocoa butter.

Compositions suitable for transdermal administration include ointments, gels and patches.

Preferably the composition is in unit dose form such as a tablet, capsule or ampoule.

Each dosage unit for oral administration contains preferably from 1 to 250 mg (and for parenteral administration contains preferably from 0.1 to 25 mg) of a compound of the formula (I) or a physiologically acceptable salt thereof calculated as the free base.

The physiologically acceptable compounds of the invention will normally be administered in a daily dosage regimen (for an adult patient) of, for example, an oral dose of between 1 mg and 500 mg, preferably between 10 mg and 400 mg, e.g. between 10 and 250 mg or an intravenous, subcutaneous, or intramuscular dose of between 0.1 mg and 100 mg, preferably between 0.1 mg and 50 mg, e.g. between 1 and 25 mg of the compound of the formula (I) or a physiologically acceptable salt thereof calculated as the free base, the compound being administered 1 to 4 times per day. Suitably the compounds will be administered for a period of continuous therapy, for example for a week or more.

The invention is further illustrated by the following non-limiting examples :

### Description 1

### 2(R,S)-Phenylazacycloheptane

To a solution of 1-aza-2-methoxy-1-cycloheptene (50.9 g) in benzene (600 ml) under argon was added phenyl magnesium bromide (300 ml of a 3M solution in ether) and the mixture was heated at reflux for 4 h. The reaction mixture was cooled (ice/water) and LiAlH₄ (16.9 g) was added portion wise. The mixture was then heated at reflux for 5 h, cooled to room temperature and poured onto crushed ice. The resultant slurry was basified with aqueous 10% sodium hydroxide and filtered through kieselguhr, which was then washed with ether. The layers were separated and the aqueous fraction was further extracted with ether. The combined ether layers were dried (sodium sulphate) and evaporated to dryness *in vacuo* to give an orange-gold liquid, which on distillation (0.5 mm Hg) gave the **title compound** as a colourless liquid (46.43 g, 66%), bp 82-90°C. ¹H NMR (CDCl₃) δ: 1.50 - 2.09 (8H, broad m), 2.75 - 2.95 (1H, m), 3.06 - 3.24 (1H, m), 3.65 - 3.81 (1H, dd, J=4,10 Hz), and 7.13 - 7.45 (5H, m).

The following compounds were prepared according to the general method of Description 1:

### 2-(R,S)-(4-Fluorophenyl)azacycloheptane

¹H NMR (CDCl₃) δ: 1.62 - 1.98 (8H, m), 2.79 - 2.89 (1H, m), 3.08 - 3.17 (1H, m), 3.71-3.80 (1H, m), 6.95 - 7.02 (2H, m), and 7.26 - 7.33 (2H, m).

### 2-(R,S)-(4-Methylphenyl)azacycloheptane

¹H NMR (CDCl₃) δ: 1.50 - 2.0 (8H, m), 2.32 (3H, s), 2.78 - 2.87 (1H, m), 3.09 - 3.17 (1H, m), 3.71 (1H, m), 7.12(2H, d, J=8 Hz), and 7.24 (2H, d, J=8 Hz)

### 2-(R,S)-(4-Methoxyphenyl)-azacycloheptane

¹H NMR (CDCl₃) δ: 1.61 - 1.97 (8H, m), 2.78 - 2.87 (1H, m), 3.09 - 3.16 (1H, m), 3.67-3.72 (1H, m), 3.79 (3H, s), 6.84 (2H, d, J=9 Hz), and 7.26 (2H, d, J=9Hz)

### Descriptions (2a) and (2b)

### N-[(S)-α-methoxyphenylacetyl]-2-(R)-phenylazacycloheptane (D2a) and N-[(S)-α-methoxyphenylacetyl)-2-(S)-phenylazacycloheptane (D2b)

To a solution of (S)-(+)-α-methoxyphenylacetic acid (3.65 g, 22 mmol) in dichloromethane (35 ml) and dimethylformamide (1 drop) was added oxalyl chloride (2.8 ml, 33 mmol), dropwise, under argon at room temperature. After 2 h the volatiles were removed *in vacuo* and the residue was dissolved in dichloromethane (90 ml). The solution was added at room temperature, with vigorous mechanical stirring, to a solution of 2-(R,S)-phenylazacycloheptane (3.88 g, 22 mmol) in dichloromethane (90 ml) and 5% aqueous NaOH (190 ml). After 0.25 h the layers were separated and the organic layer was washed with water, dried, and evaporated *in vacuo* to give a colourless oil.
Chromatography on silica gel eluting with 20-30% ethyl acetate/pentane afforded the title compounds [total yield 88%] as two diastereoisomers:
the faster eluting isomer (D2a) (3.18g, 45%)
¹H NMR (CDCl₃) exists as two conformers, δ: 0.80 - 1.06 (broad m) and 1.14 - 1.47 (broad m) (together 1H), 1.55 - 1.90 (6H, broad m), 2.17 - 2.40 (1H, broad m), 2.80 - 2.97 (1H, broad m), 3.14 (s) and 3.47 (s) (together 3H), 3.90 - 4.05 (broad d, J=13 Hz) and 4.29 - 4.45 (broad d, J=13 Hz) (together 1H), 4.98 (s) and 5.08 (s) (together 1H), 5.12 (q, J=5 Hz) and 5.42 (q, J=5 Hz) (together 1H), and 7.02 - 7.48 (10H, m).
and the slower eluting isomer (D2b) (3.04 g, 43%)
¹H NMR (CDCl₃) exists as two conformers, δ: 0.54 - 0.79 (broad m) and 1.10 - 1.45 (broad m) (together 1H), 1.50 - 1.98 (6H, broad m), 2.13 - 2.49 (broad m) and 2.76 - 3.10 (broad m) (together 2H), 3.18 (s) and 3.40 (s) (together 3H), 3.78 (broad d, J= 16 Hz) and 4.58 (broad d, J=16 Hz) (together 1H), 4.64 (s) and 5.03 (s) (together 1H), 4.72 (q, J=5 Hz) and 5.64 (q, J=5 Hz) (together 1H), and 6.94 - 7.50 (10H, m).

### Description 3

### 2-(R)-Phenylazacycloheptane

To a solution of the faster eluting amide diastereoisomer (D2a) (3.18 g, 9.8 mmol) in dry tetrahydrofuran (32 ml) under argon at room temperature was added methyl lithium (25.4 ml, 4 eqiv. of a 1.5 M solution in ether). After 40 min. the reaction was basified with saturated aqueous potassium carbonate and extracted into ether. The ether fraction was extracted into aqueous 5N hydrochloric acid, rebasified, and extracted into ether. It was dried (sodium sulphate) and evaporated to dryness *in vacuo* to give the crude product as a yellow oil, which was distilled using a Kugelrohr to give the **title compound** as a colourless oil (0.67g, 39%), bp 100°C (0.3 mm Hg), [α]²⁰_{D} + 35° (c, 0.89%, MeOH), ee > 92.6% by HPLC on a Chiralcel OJ column using hexane/ethanol as the eluant.
¹H NMR (CDCl₃) δ: 1.54 - 2.04 (8H, broad m), 2.78 - 2.96 (1H, m), 3.07 - 3.23 (1H, m), 3.68 - 3.84 (1H, m), and 7.18 - 7.47 (5H, m).
The stereochemistry was identified by comparison with the opposite enantiomer (product of Description 4)

### Description 4

### 2-(S)-Phenylazacycloheptane

The **title compound** was obtained in 33% yield from the slower eluting diastereoisomer (D2b) (3.04 g, 9.4 mmol) using a procedure similar to Description 3, [α]²⁰_{D} -30.8° (c, 0.9 %, MeOH), ee > 98.8% by HPLC on a Chiralcel OJ (TM) column using hexane/ethanol as the eluant.
¹H NMR (CDCl₃) δ: 1.49 - 2.10 (8H, broad m), 2.76 - 2.97 (1H, m), 3.05 - 3.23 (1H, m), 3.68 - 3.84 (1H, m), and 7.12 - 7.50 (5H, m).
The stereochemistry was confirmed by single crystal X-ray structure determination of the hydrobromide salt.

The following compounds were prepared according to the general method of Examples 2a, 2b, 3 and 4:

### N-[(S)-α-Methoxyphenylacetyl]-2-(R)-phenylpiperidine and N-[(S)-α-methoxyphenylacetyl]-2-(S)-phenylpiperidine

Faster eluting isomer (6.52 g; 31%),
¹H NMR (CDCl₃) δ: 1.22 - 1.91 (5H, broad m), 2.15 - 2.48 (1H, broad m), 2.56 - 2.79 (1H, broad t, J=13 Hz), 3.55 (3H, broad s), 3.73 - 4.00 (1H, broad m), 5.18 (1H, s), 5.82-6.11 (1H, broad m), and 7.05 - 7.62 (10H, m).
Slower eluting isomer (6.0 g, 29%), ¹H NMR (CDCl₃) exists as two conformers, δ: 1.21 - 1.63 (4H, broad m), 1.71 - 1.94 (broad m) and 2.09 - 2.22 (broad m) (together 1H), 2.28 - 2.45 (broad m) and 2.51 - 2.85 (broad m) (together 2H), 3.44 (s) and 3.56 (s) (together 3H), 3.90 (d, J=12 Hz) and 4.63 (d, J=12 Hz) (together 1H), 4.95 (s) and 5.18 (s) (together 1H), 5.18 (broad s) and 6.01 (broad s) (together 1H), and 7.02 - 7.57 (10H, m).

### 2-(R)-Phenylpiperidine

Prepared from faster eluting amide isomer. [α]²⁰_{D} +26.30° (c 0.9%, methanol), ¹H NMR (CDCl₃) δ: 1.38 - 1.90 (6H, broad m), 2.66 - 2.81 (1H, dt, J=12,3 Hz), 3.06 - 3.21 (1H, d, J=12 Hz), 3.46 - 3.58 (1H, dd, J=12.3 Hz), and 7.10 - 7.38 (5H, m).
The stereochemistry was confirmed by single crystal X-ray structure determination of the hydrobromide salt.

### 2-(S)-Phenylpiperidine

Prepared from slower eluting amide isomer. [α]²⁰_{D} -24° (c 0.9% in methanol) ¹H NMR (CDCl₃) δ: 1.33 - 1.90 (6H, broad m), 2.63 - 2.80 (1H, dt, J=12.3 Hz), 3.03 - 3.20 (1H, d, J=12 Hz), 3.44 -3.58 (1H, dt, J=10.2 Hz), and 7.09 - 7.38 (5H, m).

The stereochemistry was confirmed by comparison with the opposite enantiomer (above).

### N-[(S)-α-Methoxyphenylacetyl]-2-(R)-phenylpyrrolidine and N-[(S)-α-Methoxyphenylacetyl)-2-(S)-phenylpyrrolidine

### Faster eluting isomer (5.19 g, 43%)

¹H NMR (CDCl₃) exists as two conformers, δ: 1.70 - 1.88 (3H, broad m); 2.07 - 2.37 (1H, broad m); 3.14 (s) and 3.45 (s) (together 3H), 3.60 (broad t, J=7 Hz) and 3.77 (broad t, J=7 Hz), (together 2H), 4.69 (s) and 4.97 (s) (together 1H), 5.18 - 5.30 (1H, m), and 6.72 - 7.52 (10H, m).
Slower eluting isomer (4.17 g, 35%)
¹H NMR (CDCl₃) exists as two conformers, δ: 1.68 - 1.90 (3H, broad m), 2.07 - 2.25 (1H, m), 3.05 (s) and 3.43 (s) (together 3H), 3.50 - 3.98 (2H, m), 4.43 (s) and 4.91 (s) (together 1H), 4.74 (broad d, J=8 Hz) and 5.25 (broad d, J=8 Hz) (together 1H), and 7.1 - 7.46 (10H, m).

### (+)-2-Phenylpyrrolidine

Prepared from faster eluting amide isomer. [α]²⁰_{D} +38.2° (c 1.0% in methanol), ¹H NMR (CDCl₃) δ: 1.54 - 2.0 (3H, m), 2.08 - 2.27 (1H, m), 2.9 - 3.08 (1H, m), 3.12 - 3.28 (1H, m), 4.10 (1H, t, J=8 Hz), and 7.12 - 7.42 (5H, m).

### (-)-2-Phenylpyrrolidine

Prepared from slower eluting amide isomer. [α]²⁰_{D} -24.9° (c 0.9% in methanol) ¹H NMR (CDCl₃) δ: 1.53 - 1.93 (3H, m), 2.00 - 2.20 (1H, m), 2.84 - 3.02 (1H, m), 3.08 - 3.22 (1H, m), 4.03 (1H, t, J=7 Hz), and 7.08 - 7.40 (5H, m).

### Description 5

### N-Formyl-2-(R,S)-phenylazacycloheptane

To a mixture of, 98-100% formic acid (30 ml) and acetic anhydride (100 ml) was added 2-(R,S)-phenylazacycloheptane, (10 g, 57.1 mmol) and the mixture warmed to 70°C for 2 h. The reaction mixture was then cooled and evaporated to dryness *in vacuo* and the residue was partitioned between ether and saturated aqueous potassium carbonate. The ether layer was separated and evaporated to dryness *in vacuo* to afford the title compound (11.24 g; 97%) as a mixture of E/Z isomers.
¹H NMR (CDCl₃) exists as two conformers; δ 1.2-2.1(7H, m), 2.3-2.6 (1 H, m), 2.8 (t, J=12 Hz) and 3.3 (t, J=12 Hz) (together 1H), 3.65 (broad d, J=12 Hz) and 4.24 (broad d, J=12 Hz) (together 1H), 4.67 (q, J=7 Hz) and 5.3 (q, J=7 Hz) (together 1H), 7.13-7.45, (5H, m), 8.15 (s) and 8.3 (s) (together 1H).

The following compounds were prepared according to the general method of Description 5:

### N-Formyl-2-(R)-phenylazacycloheptane

¹H NMR (CDCl₃) exists as two conformers, δ: 1.19 - 2.09 (7H, m), 2.39 (m) and 2.57 (m) (together 1H), 2.81 (t, J=13 Hz) and 3.34 (t, J=13 Hz) (together 1H), 3.68 (broad d, J=13 Hz) and 4.24 (broad d, J=13 Hz) (together 1H), 4.68 (q, J=7 Hz) and 5.31 (q, J=7 Hz) (together 1H), 7.13 - 7.50 (5H, m), 8.15 (s) and 8.29 (s) (together 1H).

### N-Formyl-2-(S)-phenylazacycloheptane

¹H NMR (CDCl₃) exists as two conformers, δ: 1.26 - 2.12 (7H, m), 2.36 (m) and 2.57 (m) (together 1H), 2.81 (dt, J=13,1 Hz) and 3.34 (dt, J=13,1 Hz) (together 1H), 3.70 (broad d, J=13 Hz) and 4.25 (broad d, J=13 Hz) (together 1H), 4.69 (q, J=5 Hz) and 5.32 (q, J=5 Hz) (together 1H), 7.13 - 7.45 (5H, m), 8.16 (s) and 8.29 (s) (together 1H).

### N-Formyl-2-(R,S)-(4-fluorophenyl)azacycloheptane

¹H NMR (CDCl₃) δ: 1.27 - 1.54 (2H, m), 1.62 - 1.95 (5H, m), 2.27 - 2.38 (m) and 2.47 - 2.59 (m) (together 1H), 2.72 - 2.83 (m) and 3.25 - 3.35 (m) (together 1H), 3.64- 3.76 (m) and 4.19 - 4.24 (m) (together 1H), 4.62 - 4.69 (m) and 5.23 - 5.30 (m) (together 1H), 6.95 - 7.10 (2H, m), 7.13 - 7.33 (2H, m), and 8.14 (s) and 8.26 (s) (together 1H).

### Description 6

### 2-Methoxy-5-(N,N-di-n-propylaminosulphonyl)benzoic acid

To a solution of di-n-propylamine (25 ml) in water (200 ml), cooled to 10 °C, was added in portions 5-chlorosulphonyl-2-methoxybenzoic acid [prepared as described by W. Liebenow et al. Ger. Offen 2,721,643 *(Chem Abs.* **90**, 103673j)] (15g, 0.06 mol). The solution was stirred for 1 h, and then acidified with 5N hydrochloric acid. The resulting product was filtered, washed with hexane and recrystallised from ethyl acetate/hexane to give the **title compound** (9g; 50 %) as colourless microcrystals, mp 105 °C, (Found C, 52.93; H, 6.45; N, 4.49. C₁₄H₂₁NO₅S requires C, 53.32; H, 6.71; N, 4.44%).

The following compounds were prepared by the general method of Description 6:
5-(N,N-dimethylaminosulphonyl)-2-methoxybenzoic acid, mp 138-140°C
5-(N,N-diethylaminosulphonyl)-2-methoxybenzoic acid, mp 112-3°C
2-methoxy-5-(1-pyrrolidinylsulphonyl)benzoic acid, mp 145-146°C (ethyl acetate)
2-methoxy-5-(1-piperidinylsulphonyl)benzoic acid, mp 156-158°C,
2-methoxy-5-(4-morpholinylsulphonyl)benzoic acid, mp 181-183°C
2-methoxy-5-(4-thiomorpholinylsulphonyl)benzoic acid, mp 182-185°C
5-(1-azetidinylsulphonyl)-2-methoxybenzoic acid. mp 175-176°C
5-[(bis-(2-methoxyethyl))aminosulphonyl]-2-methoxybenzoic acid
¹H NMR (CDCl₃) δ: 3.3 (6 H, s), 3.45 (4 H, t, J = 5.4 Hz), 3.55 (4 H, t, J = 5.4 Hz), 4.13 (3 H, s), 7.15 (1H, d, J = 8.8 Hz), 8.05 (1H, d,d, J = 8.8, 2.4 Hz), and 8.58 (1H, d, J = 2.4 Hz).

### Description 7

### 3-{3-[2-(1,3 Dioxolanyl)]propionyl}-4-methoxy-N,N-dimethylbenzenesulphonamide

5-(N,N-Dimethylaminosulphonyl)-2-methoxybenzoic acid (6.89 g; 26.6 mmol) in dichloromethane (50 ml) was treated with oxalyl chloride (5.0 ml; 53.2 mmol) and one drop of dimethylformamide under an atmosphere of argon with continous stirring for 1 h. The solution was evaporated *in vacuo* and azeotroped with toluene (2x 50 ml). Trituration with hexane gave the acid chloride (6.8 g; 87%) as a colourless solid used without further purification. To dry magnesium (0.83 g, 34.6 mmol) in anhydrous tetrahydrofuran (10 ml) under an atmosphere of argon was added, dropwise, 2-(2-bromoethyl)-1,3-dioxolane (5.30 g, 29.3 mmol) at such a rate to maintain the temperature between 27°C and 30°C. After addition was complete the mixture was allowed to stir for 0.5 h at ambient temperature. This mixture was transfered dropwise over 0.5 h to anhydrous cuprous bromide (4.2g 29.3 mmol) at 0°C under an atmosphere of argon. The resulting dark mixture was stirred at 0°C for 0.75 h, and then cooled to -75°C. A solution of the dried acid chloride (above) in tetrahydrofuran (50 ml) was added dropwise at -75°C--67°C over 0.5 h. The final mixture was stirred for 1 h. at -75°C , allowed to reach ambient temperature over 0.75 h, and stirred for a further 1 h. The mixture was poured into 10% aqueous citric acid (200 ml) and ethyl acetate (200 ml) and stirred for 0.2 h. The mixture was allowed to settle and then was filtered through kieselguhr. The filtrate was separated and the aqueous layer was further extracted with ethyl acetate (3x 100 ml). The combined organic extracts were dried over sodium sulphate, filtered and evaporated *in vacuo* to a thick gum. The gum was extracted with dichloromethane (100 ml ) and filtered. The filtrate was evaporated *in vacuo* to an oil, which was chromatographed on silica, eluting with 1-20% ethyl acetate in dichoromethane to give the **title compound** as colourless microcrystals (5.17g; 66%), mp 114-115°C, (Found: C, 52.15; H, 5.99; N,4.08. C₁₅H₂₁NO₆S requires C, 52.47; H, 6.16; N,4.08%), *m/z* 343.1089. C₁₅H₂₁NO₆S requires M⁺ 343.1089

### Description 8

### 4-Methoxy-3-(1,4-dioxobutyl)-N,N-dimethylbenzenesulphonamide

A solution of 3-{3-[2-(1,3-dioxolanyl)]propionyl)-4-methoxy-N,N-dimethylbenzenesulphonamide (1.72 g, 5 mmol) was dissolved in tetrahydofuran (10 ml) and 2N hydrochoric acid (10 ml) and heated at reflux for 2 h under an atmosphere of argon. The mixture was diluted with water (50 ml) and extracted with ethyl acetate (3x 100 ml). The combined organic extracts were washed with water (10 ml), brine (50 ml) and dried over sodium sulphate. The dried extract was filtered and evaporated *in vacuo to* give the **title compound** as an oil. (1.45 g; 99%), which was used without further purification.

### Description 9

### 2-[2-Methoxy-5-(N,N-dimethylaminosulphonyl)phenyl]-1H-pyrrole

4-Methoxy-3-(1,4-dioxobutyl)-N,N-dimethylbenzenesulphonamide (1.45 g; 5 mmol) was added to ethanol (20 ml) containing ammonium acetate (2g) and heated at reflux under an atmosphere of argon for 2 h. The mixture was evaporated to one quarter volume, diluted with water (20 ml ), saturated aqueous potassium carbonate (20 ml), and extracted with ethyl acetate (3 x 80 ml). The combined extracts were washed with water (20 ml), brine (50 ml) and dried over sodium sulphate. The dried extracts were filtered and evaporated *in vacuo* to give a low melting solid. Chromatography on silica, eluting with dichloromethane, gave the title compound (0.72 g; 50 %), as colourless microcrystals, mp 129-130°C, (Found: C, 55.73; H, 5.73; N, 9.87. C₁₃H₁₆N₂O₃S requires C, 55.70; H, 5.75; N, 9.99%).

The following compounds were prepared according to the general methods of Descriptions 7-9:

### 2-(5-N,N-Diethylaminosulphonyl-2-methoxyphenyl-)-1H-pyrrole

mp 139-140°C (ether), (Found: C, 58.30; H, 6.39; N, 9.15. C₁₅H₂₀N₂O₃S requires C, 58.42; H, 6.54; N,9.08%).

### 2-[2-Methoxy-5-(1-pyrrolidinyl)sulphonylphenyl)-1H-pyrrrole

mp 118-121°C (ether), Found: C, 58.76; H, 5.87; N, 9.13. C₁₅H₁₈N₂O₃S requires C, 58.80; H, 5.92; N, 9.14%, *m/z* 306.1044. C₁₅H₁₈N₂O₃S requires M⁺ 306.1064

### 2-(5-(1-Piperidinyl)sulphonyl-2-methoxyphenyl)-1H-pyrrole

mp 137-139°C (ether), Found: C, 59.86; H, 6.25; N, 8.67. C₁₆H₂₀N₂O₃S requires C, 59.98; H, 6.29; N,8.74%, *m/z* 320.1212. C₁₆H₂₀N₂O₃S requires M⁺ 320.1230)

### 2-(5-Isopropylsulphonyl-2-methoxyphenyl)-1H-pyrrole

Mass spectrum: Found M⁺279.0934; C₁₄H₁₇NO₃S requires M⁺ 279.0929

### Description 10

### 2-(2-(2-Methoxy-5-(N,N-di-n-propylamino)sulphonyl)benzoyl)ethyl-1-3-dioxane

A solution of 2-methoxy-5-(N,N-di-n-propylaminosulphonyl)benzoic acid (7.4 g, 2.35 mmol) in anhydrous dichloromethane (50 ml), under an atmosphere of argon, was treated with oxalyl chloride (4.0 ml) and dimethylformamide (3 drops). The mixture was allowed to stir for 1 h and then was evaporated to dryness *in vacuo.* The resulting oil was dissolved in tetrahydrofuran (120 ml), under argon, and cooled to -70°C. A solution of 0.89 M 2-(2-bromomagnesiumethyl)-1,3-dioxane (43 ml, 2.35 mmol) (prepared from addition of 2-(2-bromoethyl)-1,3-dioxane to magnesium in tetrahydrofuran at reflux) was added dropwise over 0.5 h. The mixture was stirred at -70°C for a further 0.5 h, and then then allowed to warm up to ambient temperature over the next 1.5 h. The mixture was cooled to -10°C, and 10% aqueous citric acid (100 ml) was added dropwise. The resulting mixture was partitioned between water (100ml) and dichloromethane (150 ml). The aqueous layer was further extracted with dichloromethane (2x 100 ml) and the combined organic layers were washed with water (100 ml), 1.0 M NaOH (100 ml), water (100 ml), brine (100 ml ) and then dried over sodium sulphate. The dried extracts were filtered and evaporated to dryness *in vacuo* to yield the **title compound** as an oil (7.2g; 81% ), which was used without further purification.

### Description 11

### 3-(2-Methoxy-5-(N,N-di-n-propylaminosulphonyl)benzoyl)-1,1-dimethoxypropane

A solution of 2-(2-(2-methoxy-5-(N,N-di-n-propylamino)sulphonyl)benzoyl)ethyl-1-3-dioxane in methanol (200 ml) containing p-toluenesulphonic acid (0.4 g) was heated under reflux for 2.5 h. The solution was cooled , neutralised with saturated aqueous sodium bicarbonate (50 ml) and evaporated to one quarter volume. The resulting mixture was partitioned between water (100 ml) and dichloromethane (150 ml). The aqueous layer was further extracted with dichloromethane (2x 100 ml) and the combined organic layers were washed with water (100 ml), brine (100 ml), and dried over sodium sulphate. The dried extracts were filtered and evaporated to dryness *in vacuo* to yield the **title compound** (6.8g; 98%), which was used without further purification.

### Description 12

### 3-(2-Methoxy-5-(N,N-di-n-propylaminosulphonyl)benzoyl)propanal

A solution of 3-(2-methoxy-5-(N,N-di-n-propylaminosulphonyl)benzoyl)-1,1-dimethoxy propane in tetrahydrofuran (80 ml) was treated with 1N hydrochloric acid (80 ml) and stirred at room temperature for 2 h. The solution was evaporated *in vacuo* to one quarter volume and extracted with ethyl acetate (150 ml). The organic extract was washed with water (100 ml), brine (100 ml ) and dried over sodium sulphate. The dried extracts were filtered and evaporated to dryness *in vacuo* to yield the **title compound** (6.0g; 97%), which was used without purification.

### Description 13

### 2-(2-Methoxy-5-(N,N-di-n-propylaminosulphonyl)phenyl)-1H-pyrrole

A solution of 3-(2-methoxy-5-(N,N-di-n-propylaminosulphonyl)benzoyl)propanal in tetrahydrofuran (40 ml) was added to a suspension of ammonium acetate (8.0 g) in ethanol (40 ml), under argon, and warmed to gentle reflux for 1 h and then left to stir at room temperature for 17 h. The mixture was evaporated to one quarter volume and basified with saturated aqueous potassium carbonate. The resulting mixture was partitioned between water (100ml) and dichloromethane (150 ml). The aqueous layer was further extracted with dichloromethane (2x 100 ml) and the combined organic layers were washed with water (100 ml), brine (100 ml), and dried over sodium sulphate. The dried extract was filtered and evaporated to dryness *in vacuo* to give an oil, which was chromatographed on silica, eluting with 1-2% methanol in dichloromethane to give **the title compound** (2.6 g; 36%) as a colourless crystalline solid, mp 95-96°C (ether), (Found: C, 60.51; H, 7.01; N, 8.33. C₁₇H₂₄N₂O₃S requires C, 60.69; H, 7.19; N, 8.33%), *m/z* 336.1517. C₁₇H₂₄N₂O₃S requires M⁺ 336.1512

The following compounds were prepared by the general method of Descriptions 10-13:

### 2-[5-(1-Azetidinylsulphonyl)-2-methoxyphenyl]-1H-pyrrole

mp 108-109°C (ether), Found: m/z 292.0868. C₁₄H₁₆N₂O₃S requires M⁺ 292.0882

### 2-(2-Methoxy-5-[(bis-(2-methoxyethyl))aminosulphonyl]phenyl-1H-pyrrole

mp 58-62°C (50%).
¹H NMR (CDCl₃) δ: 3.3 (6H s), 3.4 (4 H, J = 4 Hz), 3.55 (4 H, t,J = 5.4 Hz), 4.05(3 H, s), 6.03 (1 H, m), 6.70 (1 H, m), 6.90 (1 H, m), 7.03 (1 H, d, J = 8.8 Hz), 7.60 (1 H, dd, J = 8.8, 2.4 Hz), 8.08 (1 H, d, J = 2.4 Hz), and 9.75 (1H, broad s).

### 2-{5-[(N-Ethyl-N-(2-methoxyethyl)amino)sulphonyl]-2-methoxyphenyl)}-1H-pyrrole

mp 84-85°C. (Found C,56.58; H, 6.38; N, 8.45%. C₁₆H₂₂N₂O₄S requires C, 56.79; H, 6.55; N, 8.28 %), *m/z* 338.1312. C₁₆H₂₂N₂O₄S requires M⁺ 338.1299

### 2-(5-Benzylsulphonyl-2-methoxyphenyl)-1H-pyrrole

NMR δ (CDCl₃) 4.00 (3H, s), 4.30 (2H, s), 6.30 (1H, m), 6.50 (1H, m), 6.90 (1H, m), 6.95 (1H, d, J = 9Hz), 7.05-7.40 (6H, m), 7.80 (1H, d, J=1Hz), 9.60 (1H, broad s).

### 2-[5-(1,2,3,6-Tetrahydropyridine-1-sulphonyl)-2-methoxyphenyl]-1H-pyrrole

NMR δ (CDCl₃) 2.20 (2H, bm), 3.20 (2H, t, J = 6Hz), 3.60 (2H, m), 4.05 (3H, s), 5.55-5.85 (2H, m), 6.30 (1H, m), 6.70 (1H, m), 6.90 (1H, m), 7.05 (1H, d, J = 9Hz), 7.60 (1H, dd, J=1Hz, 9Hz), 8.00 (1H, d, J=1Hz), 9.75 (1H, broad s).

### 2-(2-Methoxy-5-(4-morpholinyl)sulphonylphenyl)-1H-pyrrole

mp 149-151°C (ether), Found: C,55.13; H, 5.68; N, 8.24; C₁₅H₁₈N₂O₄S .0.25H₂0 requires C, 55.13; H, 5.67; N, 8.58%, *m/z* 322. C₁₅H₁₈N₂O₄S requires M⁺ 322.

### 2-(2-Methoxy-5-(4-thiomorpholinyl)sulphonylphenyl)-1H-pyrrole

mp 162-163°C (ether), Found: C, 52.86; H, 5.30; N, 8.13. C₁₅H₁₈N₂O₃S₂ requires C, 53.23; H, 5.36; 8.28%, *m/z* 338. C₁₅H₁₈N₂O₃S₂ requires M⁺ 338.

### Description 14

### 5-[(N-Ethyl-N-(2-methoxyethyl)amino)sulphonyl]-2-methoxybenzoic acid

5-Chlorosulphonyl-2-methoxybenzoic acid (25g, 0.1 mol) was added, portionwise, to a solution of N-ethyl-N-(2-methoxyethyl)amine (prepared as described by L. H. Craine et al. *J. Am*. *Chem*. *Soc,* **105**, 7252-7255) (10.3 g, 0.1 mol) in H₂O (200 ml) containing triethylamine (28 ml), at 10 °C. The solution was stirred for 1 h and then acidified with 5N hydrochloric acid. The resulting product was filtered, washed with hexane and recrystallised from ethyl acetate/hexane to give the **title compound** (17g; 54 %) as colourless microcrystals, mp 93.5-94.5 °C. (Found: C, 49.06; H,5.82; N, 4.68 %. C₁₃H₁₉NO₆S requires C,49.20; H,6.03; N,4.41 %), *m/z* 317.0939. C₁₃H₁₉NO₆S requires M⁺ 317.0933

The following compound was prepared according to the general method of Description 14:

### 5-(1,2,3,6-Tetrahydropyridine-1-sulphonyl)-2-methoxybenzoic acid

NMR δ (CDCl₃) 2.20 (2H, bm), 3.20 (2H, t, J = 6Hz), 3.60 (2H, m), 4.15 (3H, s), 5.55-5.85 (2H, m), 7.20 (1H, d, J = 9Hz), 8.00 (1H, dd, J<1Hz, 9Hz), 8.55 (1H, d, J<1Hz).

### Description 15

### 5-Benzylsulphonyl-2-methoxybenzoic acid

To a stirred solution of sodium sulfite (17.64g, 0.14 mol) and sodium hydrogen carbonate (25.8g, 0.3 mol) in water (250 ml) at 67° C was added 5-chlorosulphonyl-2-methoxybenzoic acid (25.05g, 0.1 mol) portionwise over 5 minutes. After a further 3 hours at 65°C MeOH (100 ml) and benzyl chloride (13.8 ml, 0.12 mol) were added sequentially and the mixture stirred at 65° C for a further 20 hours, cooled and poured into water (2L). The aqueous solution was washed with CH₂Cl₂ (2 x 250ml), acidified with concentrated HCI and the resultant solid filtered, washed with water and dried to afford the **title compound** as a white solid (14.07g).
NMR δ (CDCl₃) 4.00 (3H, s), 4.30 (2H, s), 6.95 (1H, d, J = 9Hz), 7.05-7.35 (5H, m), 7.60 (1H, dd, J = 1Hz, 9Hz), 8.30 (1H, d, J = 1Hz).

The following compound was prepared by the general method of Description 15:

### 5-Isopropylsulphonyl-2-methoxybenzoic acid

NMR δ (CDCl₃) 1.30 (6H, d, J=6Hz), 3.20 (1H, m), 7.20 (1H, d, J = 9Hz), 8.10 (1H, dd, J= 1Hz, 9Hz), 8.60 (1H, d, J=1Hz).

### Description 16

### 2-(3,5-Dibromo-2-methoxyphenyl)-1H-pyrrole

Prepared from 3,5-dibromo-2-hydroxybenzoic acid by reaction with dimethylsulphate followed by conversion to the acid chloride and finally reaction according to the method of Kruse et al., (Heterocycles, 26, 3141, 1987).
NMR δ (CDCl₃) 3.75 (3 H, s), 6.32 (1 H, m), 6.60 (1 H, m), 6.93 (1 H, m), 7.49 (1 H, d, J = 2 Hz), 7.65 (1 H, d, J = 2 Hz), 9.63 (1 H, br s).

### Description 17

### 2-(3-Bromo-5-ethylsulphonyl-2-methoxyphenyl)-1H-pyrrole

Prepared from 5-ethylsulphonyl-2-methoxybenzoic acid by reaction with bromine and 49% hydrogen bromide in acetic acid to give 3-bromo-5-ethylsulphonyl-2-hydroxybenzoic acid, followed by reaction with dimethylsulphate, conversion to the acid chloride using thionyl chloride and finally reaction by the method of Kruse et al (Heterocycles, *26*, 3141, 1987).
NMR δ (CDCl₃) 1.3 (3 H, t, J = 7 Hz), 3.2 (2 H, q, J = 7 Hz), 3.8 (3 H, s), 6.35 (1 H, m), 6.75 (1 H, m), 6.95 (1 H, m), 7.9 (1 H, d, J = 7 Hz), 8.05 (1 H, d, J = 7 Hz), 9.6 (1 H, br s).

### Description 18

### 2-(2-Methoxy-5-phenylsulphonylphenyl)-1H-pyrrole

(a) Phenylsulphonyl chloride (17.7 g, 100 mmol) was added dropwise at ambient temperature to a stirred solution of methyl salicylate (15.2 g, 100 mmol) and triethylamine (14 ml, 100 mmol) in dichloromethane (300 ml). After 2 h the mixture was evaporated *in vacuo* and the residue partitioned between ether and water. The organic layer was washed with water, dried (Na₂SO₄) and evaporated *in vacuo.* The crude material was purified by chromatography on silica gel using ether - pentane to afford 2-phenylsulphonyloxybenzoic acid methyl ester. NMR δ (CDCl₃) 3.8 (3 H, s), 7.0 - 8.0 (8 H, m).
(b) 2-Phenylsulphonyloxybenzoic acid methyl ester (20.3 g, 69.5 mmol) and aluminium chloride (18.6 g, 139 mmol) were stirred together at 140°C for 1.25 h. The mixture was allowed to cool and stirred with concentrated HCl (150 ml) and ice (50 g). The product was extracted into dichloromethane (3 x 200 ml) and the combined extracts dried (Na₂SO₄) and evaporated *in vacuo.* The residue was dissolved in AR acetone (250 ml) and potassium carbonate (19.2 g, 139 mmol) added. To this mixture was added dimethyl sulphate (13 ml, 137 mmol) dropwise and the resultant mixture heated at reflux for 21 h. The cooled mixture was filtered and the filter cake washed with fresh acetone. Combined filtrates were evaporated *in vacuo* and the residue treated with water (250 ml) and NaOH (40%; 12.7 ml). The mixture was heated at reflux for 3 h. The solution was cooled and washed with dichloromethane. The aqueous layer was acidified with concentrated HCl and extracted with dichloromethane (2 x 150 ml). Combined extracts were dried (Na₂SO₄) and evaporated *in vacuo* to give 2-methoxy-5-phenylsulphonylbenzoic acid (8.8g) NMR δ (CDCl₃) 4.1 (3 H, s), 7.15 (2 H, d, J = 8 Hz), 7.4 - 7.7 (3 H, m), 7.95 (2 H, m), 8.2 (1 H, dd, J = 8 Hz, 2 Hz), 8.65 (1 H, d, J = 2 Hz).
(c) The product of part (b) was reacted with thionyl chloride and the resulting acid chloride further reacted according to the method of Kruse et al., (Heterocycles, 26, 3141, 1987) to give the **title compound**
   NMR δ (CDCl₃) 4.0 (3 H, s), 6.3 (1 H, m), 6.75 (1 H, m), 6.9 (1 H, m), 7.0 (1 H, d, J = 8 Hz), 7.4 - 7.6 (3 H, m), 7.7 (1 H, dd, J = 8 Hz, 2 Hz), 7.95 (2 H, dd, J = 8 Hz, 2 Hz), 8.2 (1 H, d, J = 2 Hz), 9.7 (1 H, br s).

### Example 1

### 2-(5-Ethylsulphonyl-2-methoxyphenyl)-5-(1-(2-(R,S)-phenylpiperidinyl)-methyl)-1H-pyrrole, oxalate

To a solution of 2-phenylpiperidine (0.14g) (prepared as described by C.G. Overberger, *J.Org.Chem.,* **27**, 417 (1962)) in ethanol (20ml) was added formaldehyde (0.07ml of a 37-40% aqueous solution). This was stirred at room temperature for 0.5h before adding glacial acetic acid (0.07ml) and stirring continued for a further 10min. A solution of 2-(5-ethylsulphonyl-2-methoxyphenyl)-1H-pyrrole (0.19g, C.G. Kruse et al., Heterocycles, vol 26, 3141, 1987) in ethanol (3.3ml) was added and the mixture stirred at room temperature for 72h. The solvent was removed *in vacuo,* the residue chromatographed on silica gel with 5% methanol-dichloromethane as eluant, and the pure fractions combined. The free base was dissolved in dichloromethane and one equivalent of oxalic acid in dichloromethane was added. This solution was evaporated *in vacuo to* give the **title compound** as a pink foam, 0.06g (17%). m.p. 107-110°
¹H NMR (CDCl₃) δ: 1.29 (3H, t, J=7Hz), 1.45-1.70 (1H, br m), 1.75-2.27 (4H, br m), 2.41-2.74 (2H, br m), 3.15 (2H, q, J=7Hz), 3.40-3.53 (1H, br d, J=13Hz), 3.62-3.73 (1H, d, J=13Hz), 3.73-3.86 (1H, d, J=13Hz), 4.00 (3H, s), 4.27-4.41(1H, d, J=13Hz), 6.25 (1H, m), 6.63-6.74 (1H, m), 7.06 (1H, d, J=7Hz), 7.37-7.64 (5H, br m), 7.64-7.78 (1H, dd, J=8,2Hz), 8.25 (1H, d, J=2Hz), 11.37-11.53 (1H br s)

The following compound was prepared according to the general method of Example 1:
**(a) 2-(5-N,N-Diethylaminosulphonyl-2-methoxyphenyl)-5-[1-(2-(R,S)-phenylazacycloheptyl)methyl]-1H-pyrrole**
   mp 94-96°C, *m/z* 495.2540. C₂₈H₃₇N₃O₃S requires M⁺ 495.2556.
   ¹H NMR (CDCl₃) δ: 1.13 (6H, t, J=7 Hz), 1.50 - 2.00 (7H, broad m), 2.75 (1H, m), 2.98 (1H, m), 3.23 (4H, q, J=7 Hz), 3.47-3.58 ( 2H, J=14.5 Hz), 3.73 (1H, t, J=6 Hz), 4.05 (3H, s), 6.00 (1H, t, J=3 Hz), 6.56 (1H, t, J=3 Hz), 7.01 (1H, d, J=9 Hz), 7.23 (2H, t, J=7 Hz), 7.33 (2H, t, J=6 Hz), 7.45 (2H, d, J=7 Hz), 7. 55 (1H, dd, J=7 Hz), 7.99 (1H, d, J=3 Hz) and 9.70 (1H, broad s).

### Example 2

### 2-(5-Ethylsulphonyl-2-methoxyphenyl)-5-(1-(2-(R,S)-phenylpyrrolidinyl-methyl)-1H-pyrrole, oxalate

To a solution of of 2-phenylpyrrolidine (0.882g, 0.6 mmol ) (prepared as described by J.L.Seeman *Synthesis* 498 (1977)) in ethanol (10 ml) was added, at 0°C, 0.5 g of aqueous formaldehyde (30-40% aqueous solution). This was allowed to warm to room temperature for 0.5 h before adding glacial acetic acid 0.5 g and stirred for a further 10 min. A solution of 2-(5-ethylsulphonyl-2-methoxy)-1H-pyrrole (1g, 0.4 mmol) in ethanol (20 ml) was added and the reaction was stirred for a further 24 h. The reaction was then concentrated *in vacuo to* a gum which partitioned between dichloromethane and saturated aqueous potassium carbonate. The organic layer was separated and dried over sodium sulphate and concentrated *in vacuo* to a gum which was chromatographed on silica using a gradient of 3-5% methanol in dichloromethane. The major product of the reaction eluted in 5% methanol in dichloromethane and was concentrated *in vacuo* to a gum (0.98 g; 59% from the pyrrole). The oxalate salt was prepared by adding to an ethereal solution one equivalent of oxalic acid in ether. When a solution in acetone was concentrated in high vacuum, it gave the **title compound** as a pale green foam.
¹H NMR (d₆_DMSO) δ: 1.05 (3H, t, J=7 Hz), 2.05 (4H, m, ), 3.2 (1H, m, ), 3.3 (2H, q, J=7 Hz), 3.4 (1H, m.), 4.0 (3H, s), 4.15 (2H, s), 4.35 (1H, m), 6.2 (1H, m), 6.75(1H, m) 7.2-7.5 (6H, m) 7.5 (1H, m, dd J=7 Hz, J=1Hz), 7.95 (1H, d, J=1 Hz), 11.55 (1H, s).

### Example 3

### 2-(5-Ethylsulphonyl-2-methoxyphenyl)-5-(1-(2-(R,S)-phenylazacycloheptyl)-methyl)-1H-pyrrole, hydrochloride

To a solution of 2-(R,S)-phenylazacycloheptane (0.6 g, 3.43 mmol) [prepared from caprolactam as described by B. E. Maryanoff et al. *J*. *Med. Chem.,* **30**, 1433 (1987)] in ethanol (5 ml) was added formaldehyde (0.27 ml of a 37-40% aqueous solution). This was stirred at room temperature for 0.5 h before adding glacial acetic acid (0.25 ml) and stirring was continued for a further 10 min. 2-(5-Ethylsulphonyl-2-methoxy)-1H-pyrrole (0.6 g, 2.26 mmol) was added in one portion, and the mixture stirred at room temperature for 18 h. The solvent was removed *in vacuo,* and the residue was dissolved in dichloromethane and washed with aqueous potassium carbonate solution. The organic fraction was dried over anhydrous potassium carbonate, and chromatographed on silica gel with 1% methanol-dichloromethane as eluant, and the pure fractions combined, and evaporated *in vacuo* to give the free base which was dissolved in chloroform and shaken with 2N hydrochloric acid. The chloroform layer was dried over sodium sulphate and evaporated to dryness *in vacuo.* The residue was triturated with ether to give the **title compound** as an off-white slightly hygroscopic solid, (0.65 g; 59 %), mp 142-144°C. (Found: C, 62.67; H, 6.54; N, 5.66. C₂₆H₃₂N₂O₃S.HCl.O.5H₂O requires: C, 62.69; H, 6.88; N, 5.62%.)

The hydrochloride salt was dissolved in dichloromethane and shaken with aqueous potassium carbonate solution, washed with water, dried over anhydrous potassium carbonate and evaporated to dryness *in vacuo.* The residue was triturated with ether-pentane, and dried *in vacuo,* to give the free base as a foam.

¹H NMR (CDCl₃) δ: 1.19 (3H, t, J=7 Hz), 1.40-1.90 (8H, br m), 2.72 (1H, m), 2.91 (1H, m) 3.02 (2H, q, J=7 Hz), 3.43 (1H, d, J=14 Hz), 3.53 (1H, d, J=14 Hz), 3.66 (1H, m), 4.00 (3H, s), 5.93 (1H, t, J=3 Hz), 6.52 (1H, m), 7.01 (1H, d, J=9 Hz), 7.24 (1H, m), 7.26 (2H, m),7.38 (2H, m), 7.55 (1H, m), 7.99 (1H, d, J=2 Hz), and 9.6 (1H, br. s).

The following compounds were prepared according to the general method of Example 3:
**(a) 2-(5-Ethylsulphonyl-2-methoxyphenyl)-5-[1-(2-(R,S)-(4-methylphenyl)azacycloheptyl)methyl]-1H-pyrrole, hydrochloride**
   Found: C, 62.95; H, 6.88; N, 5.38. C₂₇H₃₄N₂O₃S. HCl. 0.5 H₂O requires: C, 63.33; H, 7.09; N, 5.47%, *m/z* (CI) 465 (M-H⁺, 3%), 280 (8), 190 (100).
**(b) 2-(5-Ethylsulphonyl-2-methoxyphenyl)-5-[1-(2-(R,S)-(4-methoxyphenyl)azacycloheptyl)methyl]-1H-pyrrole, hydrochloride**
   Found: C, 61.01; H, 6.77; N, 5.21. C₂₇H₃₄N₂O₄S. HCI. 0.5 H₂O requires C, 61.40; H, 6.87; N, 5.30 %, *m/z* 482 (M⁺, 5%), 205 (40), 162 (100), 148 (62)
**(c) 2-[2-Methoxy-5-(1-pyrrolidinyl)sulphonylphenyl]-5-[1-(2-(R,S)-phenylazacycloheptyl)methyl]-1H-pyrrole**
   Found: C, 66.00; H, 6.82; N, 8.42. C₂₈H₃₅N₃O₃S 0.75 H₂0 requires C, 66.31; H, 7.25; N, 8.29% , *m/z* 493.2363. C₂₈H₃₅N₃O₃S requires M⁺ 493.2398.
   ¹H NMR (CDCl₃) δ: 1.5-1.7 (4H, m,), 1.70 - 2.00 (8H, broad m), 2.75 (1H, m), 2.98 (1H, m), 3.23 (4H, q, J=7 Hz), 3.48-3.62 (2H, J=14.5 Hz), 3.73 (1H, t, J=6 Hz), 4.05 (3H, s), 6.00 (1H, t, J=3 Hz), 6.56 (1H, t, J=3 Hz), 7.01 (1H, d, J=9 Hz), 7.23 (1H, t, J=7 Hz), 7.33 (2H, t, J=6 Hz), 7.45 (2H, d, J=7 Hz); 7. 55 (1H, dd, J=7 Hz); 7.99 (1H, d, J=3 Hz), and 9.70 (1H, broad s).
**(d) 2-(2-Methoxy-5-(4-morpholinyl)sulphonylphenyl-5-[1-(2-(R,S)-phenylazacycloheptyl)methyl]-1H-pyrrole**
   Found: C, 64.96; H, 6.70; N,8.09. C₂₈H₃₅N₃O₄S .0.5H₂0 requires C, 64.84; H, 7 .00; N, 8.10% *m/z* 509.2330. C₂₈H₃₅N₃O₄S requires M⁺ 509.2347.
   ¹H NMR (CDCl₃) δ: 1.5-1.7 (5H, m), 1.70-2.00 (4H, broad m), 2.75 (1H, m), 2.98 (5H, m), 3.48-3.62 (2H, dd, J=14.5 Hz), 3.73 (4H, t, J=6 Hz), 4.05 (3H, s), 6.00 (1H, t, J=3 Hz), 6.56 (1H, t, J=3 Hz), 7.01 (1H, d, J=9 Hz), 7.23 (1H, t, J=7 Hz), 7.33 (2H, t, J=6 Hz), 7.45 (3H, d, J= 7 Hz), 7.90 (1H, d, J=3 Hz), and 9.70 (1H, broad s).

### Example 4

### 2-(3,5-Dibromo-2-methoxyphenyl)-5-(1-(2-(R,S)-phenylazacycloheptyl)-methyl)-1H-pyrrole, hydrochloride

A solution of 2-(R,S)-phenylazacycloheptane (0.48 g, 2.74 mmol), 37% aqueous formaldehyde (0.215 ml) and glacial acetic acid (0.2 ml) was treated with 2-(3,5-dibromo-2-methoxyphenyl)-1H-pyrrole (0.6 g, 1.807 mmol) as described for Example 3, to give the **title compound** as a slightly hygroscopic white solid, (0.345 g; 33%), mp 135-138 °C.

### Example 5

### 2-(2-Methoxy-5-N,N-dimethylaminosulphonylphenyl)-5-[1-(2-(R,S)-phenylazacycloheptyl)methyl]-1H-pyrrole, hydrochloride

A solution of 2-(R,S)-phenylazacycloheptane (0.35 g, 2.0 mmol), 37% aqueous formaldehyde (0.20 ml) and glacial acetic acid (0.12 ml ) was treated with 2-(2-methoxy-5-N,N-dimethylaminosulphonylphenyl)-1H-pyrrole (0.54 g, 2.0 mmol) as described in Example (1) to give the free base of the title compound as a pink foam. A solution in chloroform was shaken with dilute hydrochloric acid (2 ml), separated, dried over sodium sulphate, and evaporated to dryness *in vacuo* to give the **title compound** as an off-white powder (0.34g; 38% ), mp 145-150°C, (Found: C, 59.86; H, 6.42; N, 8.14.
C₂₆H₃₃N₃O₃5.HCl.0.8.H₂0 requires C, 60.23; H, 6.92; N, 8.10%), *m/z* 467.2275 C₂₆H₃₃N₃O₃S requires M⁺ 467.2245

The following compound was prepared according to the general method of Example 5:
**(a) 2-[5-(1-Azetidinylsulphonyl)-2-methoxyphenyl]-5-[1-(2-(R,S)-phenylazacycloheptyl)methyl]-1H-pyrrole**
   mp 66-67 °C (ether) (Found: C, 67.31; H, 6.83; N, 8.78. C₂₇H₃₃N₃O₃S requires C, 67.61; H, 6.93; N, 8.76 %), *m/z* 479.2220 C₂₇H₃₃N₃O₃S requires M⁺ 479.2265

### Example 6

### 2-(3-Bromo-5-ethylsulphonyl-2-methoxyphenyl)-5-(1-(2-(R,S)-phenylazacycloheptyl)methyl)-1H-pyrrole, hydrochloride

A solution of 2-(R,S)-phenylazacycloheptane (0.29 g, 1.66 mmol), 37% aqueous formaldehyde (0.16 ml) and glacial acetic acid (0.1 ml) was treated with 2-(3-bromo-5-ethylsulphonyl-2-methoxyphenyl)-1H-pyrrole (0.41 g, 1.2 mmol) as described for Example 3, except the reaction was heated at 40°C for 4 days, to give the **title compound** as an off-white solid, (0.12 g; 18%).

### Example 7

### 2-(3-Bromo-5-ethylsulphonyl-2-methoxyphenyl)-5-(1-(2-(R,S)-phenylazacycloheptyl)methyl)-1H-pyrrole, oxalate

Phosphoryl chloride (0.22 ml) was added dropwise to N-formyl-2-(R,S)-phenylazacycloheptane (0.41g) at 0°C, under argon, and the reaction mixture was stirred at room temperature for 0.5 h. 1,2-Dichloroethane (23 ml) was added and the solution cooled (ice/salt) before adding a solution of 2-(3-bromo-5-ethylsulphonyl-2-methoxyphenyl)-1H-pyrrole (0.41g) in 1,2-dichloroethane (11 ml) dropwise. The reaction mixture was stirred at room temperature for 18 h, and then cooled (ice/water) and sodium borohydride (0.54g) was added portion wise. The mixture was stirred at room temperature for 2.5 h. The excess hydride was decomposed by dropwise addition of water (3.5 ml) and methanol (3.5 ml). The mixture was partitioned between water and dichloromethane and extracted into further dichloromethane. The combined organic layers were washed with sodium carbonate solution, dried (sodium sulphate), evaporated to dryness *in-vacuo* and chromatographed on silica, eluting with 1% methanol/dichloromethane. The product was treated with oxalic acid in ether to afford the **title compound** as an off-white solid (0.31 g; 42%), mp 94-96°C.
¹H NMR (d₆-DMSO, 80°C) δ: 1.13 (3H, t, J=8 Hz), 1.46 - 2.01 (8H, broad m), 2.85-3.24 (2H, m), 3.35 (2H, q, J=8 Hz), 3.71 (3H, s), 3.75 (2H, s), 4.00 (1H, t, J=5 Hz), 6.10 (1H, broad s), 6.68 (1H, t, J=2 Hz), 7.15 - 7.52 (5H, m), 7.82 (1H, d, J=2 Hz), 7.98 (1H, d, J=2 Hz), and 10.80 (1H, broad s).

The following compounds were prepared according to the general method of Example 7:
**(a) 2-[5-(bis-(2-Methoxyethyl))aminosulphonyl-2-methoxyphenyl]-5-(1-(2-(R,S)-phenylazacycloheptyl)methyl)-1H-pyrrole, oxalate**
   sintered at 58-60°C. Found: C, 58.61; H, 6.43; N, 6.25. C₃₀H₄₁N₃O₅S:1.2 (C₂H₂O₄) requires, C, 58.63; H, 6.59; N, 6.33 %.
**(b) 2-{5-[(N-Ethyl-N-2-methoxyethyl)aminosulphonyl]-2-methoxyphenyl)}-5-[1(2-(R,S)-phenylazacycloheptyl)methyl]-1H-pyrrole**
   mp 104-105°C. (Found: C, 66.04; H, 7.36; N, 8.12. C₂₉H₃₉N₃O₄S requires C, 66.26; H,7.48; N,7.99 %), *m/z* 525.2685. C₂₉H₃₉N₃O₄S requires M⁺ 525.2660.

### Examples 8a and 8b

### 2-(3-Bromo-5-ethylsulphonyl-2-methoxyphenyl)-5-(1-(2-(R)-phenylazacycloheptyl)methyl)-1H-pyrrole (E8a) and 2-(3-Bromo-5-ethylsulphonyl-2-methoxyphenyl)-5-(1-(2-(S)-phenylazacycloheptyl)methyl)-1H-pyrrole (E8b)

The **title compounds** were prepared from the free base of Example 7 (26 mg) by HPLC [Gilson autoprep HPLC system with a Chiralpak AD (TM) (250 x 20mm) column and 2.5/97.5 ethanol-hexane plus 0.1% diethylamine as eluant at 10ml/min]
The faster enantiomer (E8a) (12.8 mg) had a retention time of 38min, [α]²⁰_{D} -74.75° (c 0.99% in chloroform), ee >98% by HPLC on Chiralpak AD (TM) column, with hexane/ethanol as the eluant.
The slower enantiomer (E8b) (13.7 mg) had a retention time of 45min,
[α]²⁰_{D} +65.35°, (c 0.98 in chloroform), ee >98.2% by HPLC on Chiralpak AD column, hexane/ethanol as the eluant.

### Example 9

### 2-(3-Bromo-5-ethylsulphonyl-2-methoxyphenyl)-5-(1-(2-(S)-phenylazacycloheptyl)methyl)-1H-pyrrole, oxalate

The **title compound** (0.34g; 45%) was also prepared from 1-formyl-2-(S)-phenylazacycloheptane (0.41 g) and 2-(3-bromo-5-ethylsulphonyl-2-methoxyphenyl)-1H-pyrrole (0.41 g) using a procedure similar to that of Example 7, mp 89-91°C, [α]²⁰_{D} + 28.4° (c, 0.76% in chloroform), ee 99.0% by HPLC on a Chiralpak AD (TM) column using hexane/ethanol as the eluant,
¹H NMR (d₆-DMSO) δ: 1.15 (3H, t, J=7 Hz), 1.45 - 2.15 (8H, broad m), 2.98 - 3.13 (1H, broad m), 3.20 - 3.32 (1H, broad m), 3.40 (2H, q, J=7 Hz), 3.68 (3H, s), 3.84 (2H, broad s), 4.18 (1H, broad s), 6.20 (1H, broad s), 6.75 (1H, broad s), 7.25 - 7.34 (1H, m), 7.38 (2H, t, J=7 Hz), 7.51 (2H, d, J=7 Hz), 7.87 (1H, d, J=2 Hz), 8.01 (1H, d, J=2 Hz), and 11.30 - 11.44 (1H, broad s).

### Example 10

### 2-(5-Ethylsulphonyl-2-methoxyphenyl)-5-(1-(2-(R)-phenylazacycloheptyl)methyl)-1H-pyrrole, oxalate

To a solution of 2-(R)-phenylazacycloheptane (0.29 g) in ethanol (4 ml) was added formaldehyde (0.13 ml of a 37-40% aqueous solution). After 0.5 h, glacial acetic acid (0.13 ml) was added and stirring was continued for a further 10 min before adding a solution of 2-(5-ethylsulphonyl-2-methoxyphenyl)- 1H-pyrrole [C. G. Kruse *et al., Heterocycles,* **26**, 3141 (1987)] (0.36g) in ethanol (6 ml). This mixture was stirred at room temperature for 18 h. The solution was evaporated to dryness *in vacuo* and the residue was chromatographed on silica, eluting with ethyl acetate/pentane (1:1). Crystallisation from ether afforded the **title compound** as a pale grey solid (0.22 g; 27%). mp 94-96°C, [α]²⁰_{D} -7.3° (c 1.0% in ethanol), ee 98.7% by HPLC on a Chiralpak AD (TM) column using hexane/ethanol as the eluant.
¹H NMR (d₆-DMSO) δ: 1.10 (3H, t, J=7 Hz), 1.43 - 2.18 (8H, broad m), 2.98 - 3.13 (1H, broad m), 3.25 (2H, q, J=7 Hz), 3.25 (1H, broad s), 3.77 - 3.88 (2H, broad s), 3.97 (3H, s), 4.10 - 4.24 (1H, broad s), 4.40 - 6.00 (2H, broad s), 6.17 (1H broad s), 6.69 (1H, t, J=3 Hz), 7.32 (2H, t, J=8 Hz), 7.42 (2H, t, J=8 Hz), 7.54 (2H, d, J=8 Hz); 7.66 (1H, dd, J=8,2 Hz), 8.00 (1H, d, J=2 Hz), and 11.07 - 11.21 (1H, broad s).

### Example 11

### 2-(5-Ethylsulphonyl-2-methoxyphenyl)-5-(1-(2-(S)-phenylazacycloheptyl)methyl)-1H-pyrrole, oxalate

The title compound was prepared from 2-(S)-phenylazacycloheptane (0.35 g, 2 mmol) and 2-(5-ethylsulphonyl-2-methoxyphenyl-1H-pyrrole (0.33 g, 1.33 mmol) by the method of Example 10, as needles, mp 90-92°C, ee 92.4% by HPLC on a Chiralpak AD (TM) column using hexane/ethanol as the eluant.

### Example 12

### 2-(2-Methoxy-5-(1-piperidinyl)sulphonylphenyl)-5-[1-(2-(R,S)-phenylazacycloheptyl)methyl]-1H-pyrrole, oxalate

Phosphoryl chloride (0.33 ml, 0.54 g, 3.5 mmol) was added dropwise to 1-formyl-2-(R,S)-phenylazacycloheptane (0.65 g, 3.2 mmol) at 0°C under argon, and the mixture was stirred at room temperature for 0.5 h. 1,2-Dichloroethane (20 ml) was added and the solution was cooled (ice/salt) before adding a solution of 2-(5-(1-piperidinyl)sulphonyl-2-methoxyphenyl)-1H-pyrrole (0.5 g, 1.6 mmol) in 1,2 -dichloroethane (20 ml), dropwise. The mixture was stirred at room temperature for 18 h and then cooled to 0°C (ice), and sodium borohydride (0.66 g) was added, portionwise. The mixture was stirred at room temperature for 2 h. The excess hydride was decomposed by dropwise addition of methanol (5 ml) and water (10 ml). The mixture was partitioned between water and dichloromethane and the aqueous fraction was further extracted with dichloromethane. The combined organic fractions were dried (sodium sulphate), evaporated to dryness *in vacuo,* and chromatographed on silica gel, eluting with hexane, followed by hexane/ether (1:1) to afford the free base of the title compound (0.7 g) (86%) as an oil. This was dissolved in ether and treated with a solution of oxalic acid (0.12 g; 1 equiv ) in ether to give the **title compound** as a colourless crystalline solid, (0.37 g), mp 96-98°C, (Found: C, 61.37; H, 6.34; N, 6.81. C₂₉H₃₇N₃O₃S.C₂H₂O₄0.6H₂O requires C, 61.22; H, 6.61; N, 6.91%).

### Example 13

### 2-(2-Methoxy-5-(4-morpholinyl)sulphonylphenyl)-5-(1-(2-(R)-phenylazacycloheptyl)methyl)-1H-pyrrole, oxalate

Phosphoryl chloride (0.18 ml) was added dropwise to N-formyl-2-(R)-phenylazacycloheptane (0.34 g) at 0°C under argon, and the mixture was stirred at room temperature for 0.5 h. 1,2-Dichloroethane (20 ml) was added and the solution was cooled (ice/salt) before adding a solution of the 2-(2-methoxy-5-(4-morpholinyl)sulphonylphenyl)-1H-pyrrole (0.32 g) in 1,2-dichloroethane (9 ml) dropwise. This mixture was stirred at room temperature for 18 h. The reaction was cooled (ice/water) and sodium borohydride (0.45 g) added portion-wise. The mixture was stirred at room temperature for 2.5 h. The excess hydride was decomposed by dropwise addition of water (2.9 ml) and methanol (2.9 ml). This mixture was partitioned between water and dichloromethane and extracted into further dichloromethane. The combined organic layers were dried (sodium sulphate), evaporated to dryness *in vacuo* and chromatographed on silica, eluting with 1% methanol/dichloromethane. The free base was treated with oxalic acid in ether to give the **title compound** as an off-white solid (0.37g, 62%), mp 107-109°C (ether), ee 97.6% by HPLC on a Chiralpak AD (TM) column using hexane/ethanol as the eluant
The following compounds were prepared according to the general method of Example 13:
**(a) 2-(2-Methoxy-5-(4-morpholinyl)suphonylphenyl)-5-(1-(2-(S)-phenylazacycloheptyl)methyl)-1H-pyrrole, oxalate**
   mp 108-109°C (ether), ee 97.8% by HPLC on a Chiralpak AD (TM) column using hexane/ethanol as the eluant
   ¹H NMR (d₆-DMSO) δ: 1.49 - 1.85 (6H, broad m), 1.85 - 2.19 (2H, broad m), 2.85 (4H, broad s), 2.99 - 3.15 (1H, broad m), 3.20 - 3.34 (1H, broad m), 3.63 (4H, broad s), 3.85 (2H, broad s), 3.98 (3H, s), 4.12 - 4.24 (1H, broad s), 6.16 (1H, broad s), 6.66 (1H, t, J=4 Hz), 7.25 - 7.35 (2H, m), 7.40 (2H, t, J=7 Hz), 7.53 (3H, d, J=7 Hz), 7.83 (1H, d, J=2 Hz), 11.11 - 11.21 (1H, broad s).
**(b) 2-(2-Methoxy-5-N,N-di-n-propylaminosuphonylphenyl)-5-(1-(2-(R)-phenylazacycloheptyl)methyl)-1H-pyrrole, oxalate**
   mp 80-82°C, [α]²⁰_{D} + 20.0° (c, 0.69% in methanol)
**(c) 2-(2-Methoxy-5-N,N-di-n-propylaminosuphonylphenyl)-5-(1-(2-(S)-phenylazacycloheptyl)methyl)-1H-pyrrole, oxalate**
   mp 75-76°C, [α]²⁰_{D} -4.6° (c 0.22% in chloroform), ee 96.2% by HPLC on a Chiralpak AD column using hexane/ethanol as the eluanL

### Example 14

### 2-(2-Methoxy-5-N,N-di-n-propylaminosulphonylphenyl)-5-[1-(2-(R,S)-phenylazacycloheptyl)methyl]-1H-pyrrole

Phosphoryl chloride (0.28 ml; 3.0 mmol) was added dropwise to N-formyl-2-(R,S)-phenylazacycloheptane (0.57 g; 2.8 mmol) at 0°C under argon, and then stirred at room temperature for 0.5 h. 1,2-Dichloroethane (20 ml) was added and the solution was cooled (ice/salt) before adding a solution of 2-(2-methoxy-5-N,N-di-n-propylaminosulphonyl)phenyl)-1H-pyrrole (0.5 g, 15 mmol) in 1,2 -dichloroethane (20 ml), dropwise. The mixture was stirred at room temperature for 18 h. The reaction mixture was cooled to 0°C (ice) and sodium borohydride (0.66 g) was added portionwise. The mixture was stirred at room temperature for 2 h. The excess hydride was decomposed by dropwise addition of methanol (5 ml) and water (10 ml). The mixture was partitioned between water and dichloromethane and the aqueous fraction was further extracted with dichloromethane. The combined organic fractions were dried (sodium sulphate), evaporated to dryness *in vacuo,* and chromatographed on silica gel, eluting with hexane, followed by hexane/ether (1:1) to afford the free base of the **title compound** (0.64 g) (82%), mp 116-119°C. Found: C, 69.17; H, 7.95; N, 8.19. C₃₀H₄₁N₃O₃S requires C, 68.80; H, 7.89; N, 8.02%., *m/z* 523.2896, C₃₀H₄₁N₃O₃S requires M⁺ 523.2867

### Example 15

### 2-(2-Methoxy-5-(4-thiomorpholinyl)sulphonylphenyl)-5-[1-(2-(R,S)-phenylazacycloheptyl)methyl]-1H-pyrrole, hydrochloride

Phosphoryl chloride (0.30 ml; 0.505 g; 3.3 mmol) was added dropwise to N-formyl-2-(R,S)-phenylazacycloheptane (0.676 g; 3.3 mmol) at 0°C under argon, and the mixture was stirred at room temperature for 0.5 h. 1,2-Dichloroethane (20 ml) was added and the solution was cooled (ice/salt) before adding a solution of 2-(2-methoxy-5-(4-thiomorpholinyl)sulphonylphenyl)-1H-pyrrole (0.75 g; 2.2 mmol) in 1,2 -dichloroethane (20 ml), dropwise. The mixture was stirred at room temperature for 18 h and then cooled to 0°C (ice), and sodium borohydride (0.84 g) was added portionwise. The mixture was stirred at room temperature for 4 h. The excess hydride was decomposed by dropwise addition of methanol (10 ml) and water (20 ml). The mixture was partitioned between water and dichloromethane and the aqueous fraction was further extracted with dichloromethane. The combined organic fractions were dried (sodium sulphate), evaporated to dryness *in vacuo.* The product was dissolved in methanol (5ml) cooled in ice and concentrated HCl (3ml) was added. The mixture was stirred at room temperature for 2 h. Water (50 ml) was added and the mixture was basified with 10% sodium carbonate and extracted with dichloromethane. The organic extract was dried (sodium sulphate) and evaporated to dryness *in vacuo.* The product was chromatographed on silica gel, eluting with ether/dichloromethane (2:1) to afford the free base of the title compound (0.8 g) (68%) as an oil. A portion (0.7 g) of the product was dissolved in chloroform and then shaken with 0.5 N hydrochloric acid. The organic phase was dried (sodium sulphate) and evaporated to dryness *in vacuo* to afford a semi-solid which was triturated with ether to afford the **title compound** (0.72 g), mp 128-135°C, (Found: C, 59.49; H, 6.47; N, 7.50. C₂₈H₃₅N₃O₃S₂.HCl requires C, 59.82; H, 6.45; 7.47 %), *m/z* 525. C₂₈H₃₅N₃O₃S₂ requires M⁺ 525.

The following compounds were prepared according to the general method of Example 15:
**(a) 2-(5-Benzylsulphonyl-2-methoxyphenyl)-5-[1-(2-(R,S)-phenylazacycloheptyl) methyl]-1H-pyrrole, hydrochloride**

| | | | | |
|---|---|---|---|---|
| Found | C, 65.41, | H, 6.21, | N, 5.05%; | C₃₁H₃₄N₂O₃S.HCl.H₂O |
| requires | C, 65.41, | H, 6.55, | N, 4.92%. | |

**(b) 2-(5-Isopropylsulphonyl-2-methoxyphenyl)-5-[1-(2-(R,S)-phenylazacycloheptyl)methyl]-1H-pyrrole, hydrochloride**

| | | | | |
|---|---|---|---|---|
| Found | C, 63.18, | H, 6.97, | N, 5.55%; | C₂₇H₃₄N₂O₃S.HCl.0.5H₂O |
| requires | C, 63.32, | H, 7.09, | N, 5.47%. | |

**(c) 2-(2-Methoxy-5-phenylsulphonylphenyl)-5-[1-(2-(R,S)-phenylazacycloheptyl)methyl]-1H-pyrrole, hydrochloride**

| | | | | |
|---|---|---|---|---|
| Found | C, 65.00, | H, 6.17, | N, 5.15%; | C₃₀H₃₂N₂O₃S.HCl.H₂O |
| requires | C, 64.91, | H, 6.36, | N, 5.05%. | |

**(d) 2-[5-(1,2,3,6-Tetrahydropyridine-1-sulphonyl)-2-methoxyphenyl]-5-[1-(2-(R,S)-phenylazacycloheptyl)methyl]-1H-pyrrole, hydrochloride**
NMR δ (CDCl₃) 1.30-2.90 (10H, m), 2.95-3.70 (6H, m), 3.80-5.10 (6H, m), 5.55-5.85 (2H, m), 6.05-6.25 (1H, m), 6.50 (1H, m), 7.05 (1H, m), 7.30-7.80 (6H, m), 7.95 (1H, dd, J<1Hz, 9Hz), 11.40 (1H, bs), 11.85-12.30 (1H, m).
**(e) 2-(2-Methoxy-5-N,N-dimethylaminosulphonylphenyl)-5-[1-(2-(R,S)-(4-fluorophenyl)azacycloheptyl)methyl]-1H-pyrrole, hydrochloride**

| | | | | |
|---|---|---|---|---|
| Found: | C, 59.79; | H, 6.38; | N, 7.59. | C₂₆H₃₂FN₃O₃S. HCl |
| requires | C, 59.82; | H, 6.37; | N, 8.05 %, | |
| *m/z* 485.2170, | | | | C₂₆H₃₂FN₃O₃S |
| requires | M⁺ 485. 2196 | | | |

**(f) 2-(3 Bromo-5-ethylsulphonyl-2-methoxyphenyl)-5-[1-(2 (R,S)-(4-fluorophenyl)azacycloheptyl)methyl]-1H-pyrrole, hydrochloride**
¹H NMR δ: 1.34 (3H, t, J=8 Hz), 1.80 - 2.19 (8H, m), 2.32 (m) and 2.46 (m) (together 1H), 2.72 (m) and 3.09 (m) (together 1H), 3.15 - 3.40 (2H, m), 3.67 (1H, m), 3.79 (s) and 3.88 (s) (together 3H), 4.25 (1H, m), 4.00 (m) and 5.05 (m) (together 1H), 6.10 (broad s) and 6.23 (broad s)(together 1H), 6.62 (t, J=3 Hz) and 6.67 (t, J=3 Hz) (together 1H), 7.14 (1H, t, J=9 Hz), 7.20 (1H, t, J=9 Hz), 7.48 (1H, m), 7.73 (1H, m), 7.95 (1H, t, J=2 Hz), 8.12 (1H, m), 11.38 (broad s) and 11.85 (broad s) (together 1H), 11.75 (broad s) and 12.12 (broad s)(together 1H),
*m/z* 548.1153, C₂₆H₃₀Br F N₂O₃S requires M⁺ 548. 1173

### Example 16

### 2-(5-Ethylsulphonyl-2-methoxyphenyl)-5-(1-(2-(R)-phenylpiperidinyl)methyl)-1H-pyrrole

To a solution of 2-(R)-phenylpiperidine (0.27 g) in ethanol (4 ml) was added formaldehyde (0.13 ml of a 37-40% aqueous solution). After 0.5 h, glacial acetic acid (0.13 ml) was added and stirring was continued for a further 10 min before adding a solution of 2-(5-ethylsulphonyl-2-methoxyphenyl)-1H-pyrrole (0.36 g) in ethanol (6 ml). This mixture was stirred at room temperature for 7 days, and then evaporated to dryness *in vacuo* and the residue was chromatographed on silica, eluting with 2% methanol/dichloromethane. Trituration with methanol afforded the **title compound** as a pink solid (0.31 g, 51%), mp 187-188°C, [α]²⁰_{D} -92.4° (c 1.0% in chloroform). ¹H NMR (CDCl₃) δ: 1.28 (3H, t, J=7 Hz), 1.32 - 1.45 (1H, broad m), 1.52 - 1.85 (5H, broad m), 2.05 - 2.20 (1H, broad t, J=11 Hz), 3.10 (5H, q, J=7 Hz), 3.68 (1H, d, J=15 Hz), 4.08 (3H, s), 6.00 (1H, t, J=3 Hz), 6.60 (1H, t, J=3 Hz), 7.08 (1H, d, J=8 Hz), 7.27 (1H, t, J=7 Hz), 7.34 (2H, t, J=7 Hz), 7.42 (2H, d, J=7 Hz), 7.65 (1H, dt, J=8,2 Hz), 8.08 (1H, d, J=2 Hz), and 9.54 - 9.68 (1H, broad s).

The following compound was prepared according to the general method of Example 16:

### (a) 2-(5-Ethylsulphonyl-2-methoxyphenyl)-5-(1-(2-(S)phenylpiperidinyl)methyl)-1H-pyrrole

Prepared from 2-(S)-phenylpiperidine. mp 187-188°C, [α]²⁰_{D} +97.4° (c 0.63% in chloroform),
¹H NMR (CDCl₃) δ: 1.27 (3H, t, J=8 Hz), 1.32 - 1.45 (1H, broad m), 1.52 - 1.73 (3H, broad m), 1.73 - 1.84 (2H, broad m), 2.13 (1H, dt, J=11,3 Hz), 3.04 - 3.18 (5H, m), 3.67 (1H, d, J=15 Hz), 4.08 (3H, s), 6.00 (1H, t, J=3 Hz), 6.60 (1H, t, J=3 Hz), 7.07 (1H, d, J=9 Hz), 7.26 (1H, t, J=5 Hz), 7.35 (2H, t, J=8 Hz), 7.42 (2H, d, J=7 Hz), 7.64 (1H, dt, J=9,2 Hz), 8.06 (1H, d, J=2 Hz), and 9.52 - 9.63 (1H, broad m).

### Example 17

### (-)-2-(S-Ethylsulphonyl-2-methoxyphenyl)-5-(1-(2-phenylpyrrolidinyl)methyl)-1H-pyrrole, oxalate

To a solution of (+)-2-phenylpyrrolidine (0.124 g) in ethanol (2 ml) was added formaldehyde (0.066 ml of a 37-40% aqueous solution). After 0.5 h glacial acetic acid (0.066 ml) was added and stirring was continued for a further 10 min before adding a solution of 2-(5-ethylsulphonyl-2-methoxyphenyl)-1H-pyrrole (0.18 g) in ethanol (3 ml). The mixture was stirred at room temperature for 12 days, and then evaporated to dryness *in vacuo* and the residue was chromatographed on silica, eluting with 2% methanol/dichloromethane. The product was treated with oxalic acid in ether to give the **title compound** as a pale brown solid (0.082 g; 23%), mp 88-89°C, [α]²⁰_{D} -35.3° (c 0.57% in chloroform), ee 97.6% by HPLC on a Chiralpak AD column using hexane/ethanol as the eluant.
¹H NMR (d₆-DMSO) δ: 1.23 (3H, t, J=7 Hz), 2.00 - 2.25 (3H, broad s), 2.37 - 2.73 (2H, broad m), 3.08 - 3.28 (1H, broad m), 3.14 (2H, q, J=7 Hz), 3.53 (1H, broad m), 4.10 (3H, s), 4.20 (1H, d, J=14 Hz), 4.37 (1H, broad s), 6.35 (1H, broad s), 6.8 (1H, broad s), 7.37 - 7.70 (6H, m), 7.80 (1H, dd, J=8,2 Hz), 8.10 (1H, d, J=2 Hz), 11.34 - 11.49 (1H, broad s).

The following compound was prepared from (-)-2-phenylpyrrolidine according to the general method of Example 17:
**(a) (+)-2-(5-Ethylsulphonyl-2-methoxyphenyl)-5-(1-(2-phenylpyrrolidinyl)methyl)-1H-pyrrole, oxalate mp 96-98°C**

### Example 18

The following compounds were also prepared by the general methods exemplified above:
(a) 2-(5-dimethylaminosulphonyl-2-methoxy-3-methylphenyl)-5-[1-(2-(R,S)-phenylazacycloheptyl)methyl]-1H-pyrrole, hydrochloride Found: C, 61.82; H, 6.94; N, 7.86; C₂₇H₃₅N₃O₃S. HCl. 0.5H₂O requires C, 61.52; H, 7.08; N, 7.97%
(b) 2-(3-chloro-5-dimethylaminosulphonyl-2-methoxyphenyl)-5-[1-(2-(R,S)-phenylazacycloheptyl)methyl]-1H-pyrrole, hydrochloride. Found: C, 57.83; H, 5.88; N, 7.91; C₂₆H₃₂ClN₃O₃S.HCl requires C, 57.99; H, 6.18; N, 7.80%
(c) 2-(5-dimethylaminosulphonyl-2-methoxy-3-trifluoromethylphenyl)-5-[1-(2-(R,S)-phenylazacycloheptyl)methyl]-1H-pyrrole, hydrochloride. Found: C, 56.72; H, 5.63; N, 7.34; C₂₇H₃₂F₃N₃O₅.HCl requires C, 56.69; H, 5.81; N, 7.35%
(d) 2-(2,3-dimethoxy-5-ethylsulphonylphenyl)-5-[1-(2-(R,S)-phenylazacycloheptyl)methyl]-1H-pyrrole, hydrochloride.
   ^{m}/z 482.2238. C₂₇H₃₄N₂O₄S requires M⁺ 482.2273
(e) 2-(3-tert-butyl-5-dimethylaminosulphonyl-2-methoxyphenyl)-5-[-(2-(R,S)-phenylazacycloheptyl)methyl]-1H-pyrrole, hydrochloride. ^{m}/z 523.2854. C₃₀H₄₁N₃O₃S requires M⁺ 523.2867
(f) 2-(5-ethylsulphonyl-3-ethylthio-2-methoxyphenyl)-5-[1-(2-(R,S)-phenylazacycloheptyl)methyl]-1H-pyrrole, mp 133.5-136.5°C. Found: C, 65.36; H, 6.75; N, 5.49; C₂₈H₃₆N₂O₃S₂ requires C, 65.59; H, 7.08; N, 5.46%
(g) 2-(2,3-dimethoxy-5-dimethylaminosulphonylphenyl)-5-[1-(2-(R,S)-phenylazacycloheptyl)methyl]-1H-pyrrole, hydrochloride. NMR δ (CDCl₃): 1.30-2.60 (8H, m), 2.70-2.90 (7H, m), 3.00-3.80 (3H, m), 3.85-4.10 (6H, m), 4.15-5.05 (1H, m), 6.05-6.25 (1H, m), 6.50-6.65 (1H, m), 7.05 (1H, d, J=2Hz), 7.30-7.55 (4H, m), 7.55-7.65 (1H, m), 7.75 (1H, m), 11.20-11.45 (1H, m), 11.80-12.20 (1H, m)
(h) 2-(3-tert-butyl-5-ethylsulphonyl-2-methoxyphenyl)-5-[1-(2-(R,S)-phenylazacycloheptyl)methyl]-1H-pyrrole, hydrochloride. Found: C, 65.12; H, 7.27; N, 5.13; C₃₀H₄₀N₂O₃S.HCl. 0.5H₂O requires C, 65.02; H, 7.46; N, 5.05%
(i) 2-(5-bromo-2,3-dimethoxyphenyl)-5-[1-(2-(R,S)-phenylazacycloheptyl)methyl]-1H-pyrrole, hydrochloride ^{m}/z (m-1⁺) 467.1357 C₂₅H₂₈N₂O₂Br requires 467.1333
(j) 2-[(2,3-dihydro-5-dimethylaminosulphonyl)benzofuran-7-yl]-5-[1-(2-(R,S)-phenylazacycloheptyl)methyl]-1H-pyrrole, hydrochloride. NMR δ (CDCl₃) 1.30-2.60 (7H, m), 2.75 (6H, s), 2.90-3.40 (2H, m), 3.30 (2H, t, J=8Hz), 3.40-4.00 (2H, m), 4.20 (1H, m), 4.70-5.05 (3H, m), 6.05-6.25 (1H, m), 6.55 (1H, m), 7.30-7.55 (5H, m), 7.65-7.85 (2H, m), 11.20-11.55 (1H, m), 11.80-12.25 (1H, m)
(k) 2-{5-[(N-isopropyl-N-methyl)aminosulphonyl]-2-methoxyphenyl}-5-[1-(2-(R,S)-phenylazacycloheptyl)methyl]-1H-pyrrole, oxalate m.p. 95-98°C observed M⁺ = 495.2568 C₂₈H₃₇N₃O₃S requires 495.2581.
(l) 2-{5-[(N-cyclopropyl-N-methyl)aminosulphonyl]-2-methoxyphenyl}-5-[1-(2-(R,S)-phenylazacycloheptyl)methyl]-1H-pyrrole, oxalate m.p. 97-98°C
(m) 2-{3-bromo-5-[(N,N-dimethylaminosulphonyl]-2-methoxyphenyl}-5-[1-(2-(R,S)-phenylazacycloheptyl)methyl]-1H-pyrrole m.p. 49-51°C. Found: C, 57.28; H, 5.89; N, 7.69. C₂₆H₃₂BrN₃O₃S requires C, 57.14; H, 5.90; N, 7.69%.
(n) 2-(5-Nitro-2-methoxyphenyl)-5-(1-(2-(R,S)-phenylazacycloheptyl)methyl)-1H-pyrrole, mp 129-130°C. Found: C, 70.59; H, 6.66; N, 10.42; C₂₄H₂₇N₃O₃. 0.25H₂O requires C, 70.31; H, 6.76; N, 10.25%
(o) 2-(5-Ethylsulfonyl-2-methoxyphenyl)-5-(1-(2-(R,S)-(4-methylphenyl)pyrrolidinyl)methyl)-1H-pyrrole, hydrochloride. Found: C, 60.91; H, 6.44; N, 5.67; C₂₅H₃₀N₂O₃S. HCl.H₂O requires C, 60.90; H, 6.75; N, 5.68%

### DATA

The ability of the compounds to bind selectively to human D₃ dopamine receptors can be demonstrated by measuring their binding to cloned receptors. The inhibition constants (Kᵢ) of test compounds for displacement of [¹²⁵I] iodosulpride binding to human D₂ and D₃ dopamine receptors expressed in CHO cells have been determined. The cell lines were shown to be free from bacterial, fungal and mycoplasmal contaminants, and stocks of each were stored frozen in liquid nitrogen. Cultures were grown as monolayers or in suspension in standard cell culture media. Cells were recovered by scraping (from monolayers) or by centrifugation (from suspension cultures), and were washed two or three times by suspension in phosphate buffered saline followed by collection by centrifugation. Cell pellets were stored frozen at -40°C. Crude cell membranes were prepared by homogenisation followed by high-speed centrifugation, and characterisation of cloned receptors achieved by radioligand binding.

### Preparation of CHO cell membranes

Cell pellets were gently thawed at room temperature, and resuspended in about 20 volumes of ice-cold 50 mM Tris salts (pH 7.4 @ 37°C), 20mM EDTA, 0.2 M sucrose. The suspension was homogenised using an Ultra-Turrax (TM) at full speed for 15 sec. The homogenate was centrifuged at 18,000 r.p.m for 20 min at 4°C in a Sorvall RC5C centrifuge. The membrane pellet was resuspended in ice-cold 50 mM Tris salts (pH 7.4 @ 37°C), using an Ultra-Turrax (TM), and recentrifuged at 18,000 r.p.m for 15 min at 4°C in a Sorvall RC5C. The membranes were washed two more times with ice-cold 50 mM Tris salts (pH 7.4 @ 37°C). The final pellet was resuspended in 50 mM Tris salts (pH 7.4 @ 37°C), and the protein content determined using bovine serum albumin as a standard (Bradford, M. M. (1976) Anal. Biochem. **72**, 248-254).

### Binding experiments on cloned dopamine receptors

Crude cell membranes were incubated with 0.1 nM [¹²⁵I] iodosulpride (∼2000 Ci/mmol; Amersham, U. K.), and the test compound in a buffer containing 50 mM Tris salts (pH 7.4 @ 37°C), 120 mM NaCl, 5 mM KCl, 2 mM CaCl₂, 1 mM MgCl₂, 0.1% (w/v) bovine serum albumin, in a total volume of 1 ml for 30 min at 37°C. Following incubation with the test compound, samples were filtered using a Brandel Cell Harvester (TM), and washed three times with ice-cold 50 mM Tris salts (pH 7.4 @ 37°C), 120 mM NaCl, 5 mM KCl, 2 mM CaCl₂, 1 mM MgCl₂. The radioactivity on the filters was measured using a Cobra (TM) gamma counter (Canberra Packard). Non-specific binding was defined as the radioligand binding remaining after incubation in the presence of 100 µM iodosulpride. For competition curves, 14 concentrations (half-log dilutions) of competing cold drug were used.

Competition curves were analysed simultaneously whenever possible using non-linear least-squares fitting procedures, capable of fitting one, two or three site models. The compounds of Examples 3, 6, 7, 8, 10 11 and 13 had IC₅₀ values of between 1 and 50 nM at the human D₃ receptor.

### Pharmaceutical Formulations

The following represent typical pharmaceutical formulations according to the present invention, which may be prepared using standard methods.

| **IV Infusion** | |
|---|---|
| Compound of formula (I) | 1-40 mg |
| Buffer | to pH ca 7 |
| Solvent/complexing agent | to 100 ml |

| **Bolus Injection** | |
|---|---|
| Compound of formula (I) | 1-40 mg |
| Buffer | to pH ca 7 |
| Co-Solvent | to 5 ml |

- Buffer:: Suitable buffers include citrate, phosphate, sodium hydroxide/hydrochloric acid.
- Solvent:: Typically water but may also include cyclodextrins (1-100 mg) and co-solvents such as propylene glycol, polyethylene glycol and alcohol.

| **Tablet** | |
|---|---|
| Compound | 1 - 40 mg |
| Diluent/Filler * | 50 - 250 mg |
| Binder | 5 - 25 mg |
| Disentegrant * | 5 - 50 mg |
| Lubricant | 1 - 5 mg |
| Cyclodextrin | 1 - 100 mg |

| | |
|---|---|
| * may also include cyclodextrins | |

- Diluent:: e.g. Microcrystalline cellulose, lactose, starch
- Binder :: e.g. Polyvinylpyrrolidone, hydroxypropymethylcellulose
- Disintegrant :: e.g. Sodium starch glycollate, crospovidone
- Lubricant:: e.g. Magnesium stearate, sodium stearyl fumarate.

| **Oral Suspension** | |
|---|---|
| Compound | 1 - 40 mg |
| Suspending Agent | 0.1 - 10 mg |
| Diluent | 20 - 60 mg |
| Preservative | 0.01 - 1.0 mg |
| Buffer | to pH ca 5 - 8 |
| Co-solvent or solubiliser | 0 - 40 mg |
| Flavour | 0.01 - 1.0 mg |
| Colourant | 0.001 - 0.1 mg |

- Suspending agent :: e.g. Xanthan gum, microcrystalline cellulose
- Diluent:: e.g. sorbitol solution, typically water
- Preservative :: e.g. sodium benzoate
- Buffer:: e.g. citrate
- Co-solvent :: e.g. alcohol, propylene glycol, polyethylene glycol
- Solubiliser:: e.g. cyclodextrin

## Claims

1. A compound of formula (I): 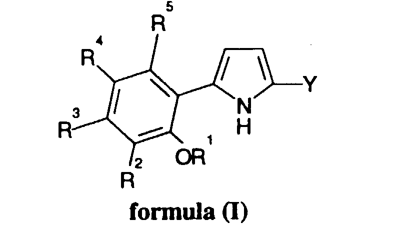 wherein
R¹ represents C₁₋₄alkyl; and
R², R³, R⁴ and R⁵ each independently represent hydrogen, halogen, C₁₋₄alkyl,
C₁₋₄alkoxy, C₁₋₄alkoxyC₁₋₄alkyl, C₁₋₄alkylsulphonyl, trifluoromethylsulphonyl; optionally substituted arylsulphonyl, optionally substituted heteroarylsulphonyl, optionally substituted aralkylsulphonyl, optionally substituted heteroarakylsulphonyl, nitro, cyano, amino, mono- or di-alkylamino,
trifluoromethyl, trifluoromethoxy, hydroxyl, hydroxyalkyl, C₁₋₄alkylthio,
C₁-₄alkanoyl, C₁₋₄alkoxycarbonyl, or -SO₂NR⁶R⁷ wherein R⁶ and R⁷ each independently represent hydrogen, C₁₋₄alkyl or C₁₋₄alkoxyC₁₋₄alkyl, or NR⁶R⁷ forms a 3- to 8- membered fully saturated heterocyclic ring, a 5- to 8-membered partially saturated heterocyclic ring, or a 5- to 8-membered fully saturated heterocyclic ring which contains in addition to the nitrogen atom an oxygen or sulphur atom; or
R¹ and R² together form a linking chain -(CH₂)ₘOₚ;
(wherein m is 2 to 4 and p is zero or 1) which chain may be optionally substituted by one or two C₁₋₄alkyl groups; and
Y represents a group of formula : 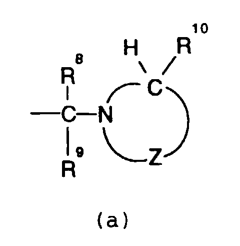
wherein
R⁸ and R⁹ independently represent hydrogen, C₁₋₆alkyl, optionally substituted arylC₁₋₆alkyl or optionally substituted heteroarylC₁₋₆alkyl;
R¹⁰ represents an optionally substituted aryl or optionally substituted heteroaryl group; and
Z represents -(CH₂)ₙ wherein n is 2 to 8 or -(CH₂)_{q}CH=CH(CH₂)ᵣ where q and r independently represent 1 to 3;
or a salt thereof.

2. A compound according to claim 1 wherein R¹ represents methyl or ethyl.

3. A compound according to claim 1 or claim 2 wherein at least one of R² to R⁵ is hydrogen, and the other substituents are selected from halogen, C₁₋₂alkyl, C₁₋₂alkoxy, C₁₋₄alkylsulphonyl, phenylsulphonyl, benzylsulphonyl and -SO₂NR⁶R⁷ wherein R⁶ and R⁷ represent C₁₋₄alkyl or C₁₋₂alkoxyC₁₋₂alkyl, or NR⁶R⁷ represents a ring selected from azetidinyl, pyrrolidinyl, piperidinyl, azacycloheptyl, 1,2,3,6-tetrahydropyridinyl, 4-morpholinyl or 4-thiomorpholinyl.

4. A compound according to any of claims 1 to3 wherein R⁸ represents hydrogen and R⁹ represents hydrogen or methyl.

5. A compound according to any of claims 1 to 4 wherein Z represents -(CH₂)ₙ wherein n is 3, 4 or 5, or -(CH₂)_{q}CH=CH(CH₂)ᵣ where the sum of q and r is 2 or 3.

6. A compound according to any of claims 1 to 5 wherein R¹⁰ represents optionally substituted phenyl.

7. A compound according to any of claims 1 to 6 wherein the group Y represents 2-phenylpyrrolidinyl, 2-phenylpiperidinyl, 2-phenylazacycloheptyl or 2-phenylazacyclooctyl.

8. A compound according to any one of claims 1 to 7 in which the carbon atom bearing R¹⁰ is of the R configuration.

9. A compound according to claim 1 selected from:
2-(5-ethylsulphonyl-2-methoxyphenyl)-5-(1-(2-(R,S)-phenylazacycloheptyl)-methyl)-1H-pyrrole,
2-(2-methoxy-5-N,N-dimethylaminosulphonylphenyl)-5-[1-(2-(R,S)-phenylazacycloheptyl)methyl]- 1H-pyrrole,
2-(3-bromo-5-ethylsulphonyl-2-methoxyphenyl)-5-(1-(2-(R,S)-phenylazacycloheptyl)methyl)- 1H-pyrrole,
2-(5-ethylsulphonyl-2-methoxyphenyl)-5-(1-(2-(R)-phenylazacycloheptyl)methyl)-1H-pyrrole,
2-(5-ethylsulphonyl-2-methoxyphenyl)-5-(1-(2-(S)-phenylazacycloheptyl)methyl)-1H-pyrrole,
2-(3-bromo-5-ethylsulphonyl-2-methoxyphenyl)-5-(1-(2-(R)-phenylazacycloheptyl)methyl)-1H-pyrrole,
2-(3-bromo-5-ethylsulphonyl-2-methoxyphenyl)-5-(1-(2-(S)-phenylazacycloheptyl)methyl)-1H-pyrrole,
2-(2-methoxy-5-dimethylaminosulphonylphenyl)-5-[1-(2-(R,S)-(4-fluorophenyl)azacycloheptyl)methyl]-1H-pyrrole,
2-(3-bromo-5-ethylsulphonyl-2-methoxyphenyl)-5-[1-(2-(R,S)-(4-fluorophenyl)azacycloheptyl)methyl]-1H-pyrrole,
2-(5-ethylsulphonyl-2-methoxyphenyl)-5-[1-(2-(R,S)-(4-methylphenyl)azacycloheptyl)methyl]-1H-pyrrole),
2-(2-methoxy-5-(4-morpholinyl)sulphonylphenyl)-5-[1-(2-(R,S)-phenylazacycloheptyl)methyl]-1H-pyrrole,
2-(2-methoxy-5-(4-morpholinyl)sulphonylphenyl)-5-(1-(2-(R)-phenylazacycloheptyl)methyl)-1H-pyrrole,
2-(2-methoxy-5-(4-morpholinyl)suphonylphenyl)-5-(1-(2-(S)-phenylazacycloheptyl)methyl)-1H-pyrrole,
2-[5-(azetidinylsulphonyl)-2-methoxyphenyl]-5-[1-(2-(R,S)-phenylazacycloheptyl)methyl]-1H-pyrrole, and
2-(5-isopropylsulphonyl-2-methoxyphenyl)l-(2-(R,S)-phenylazacycloheptyl)methyl]-1H-pyrrole,
or a salt thereof.

10. A process for preparing a compound of formula (I) as defined in any of claims 1 to 9 which process comprises :
(a) carrying out a Mannich reaction with a compound of formula (II): 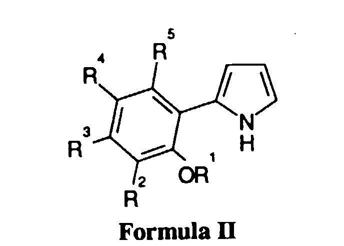 (wherein R¹-R⁵ are as hereinbefore defined),
and an amine of formula (III): 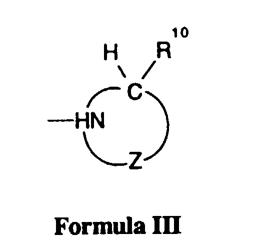 wherein R¹⁰ and Z are as hereinbefore defined;
(b) carrying out a Vilsmeier reaction with a compound of formula (II) and an amide of formula (IV): 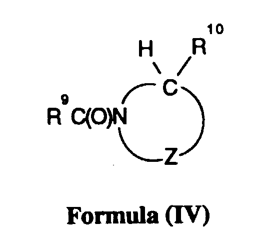 wherein R⁹, R¹⁰ and Z are as hereinbefore defined;
and reducing the intermediate product with, for example, sodium borohydride or cyanoborohydride;
c) to prepare a compound of formula (I) in which R⁸ and R⁹ both represent hydrogen, reductive amination of a compound of formula (V) : 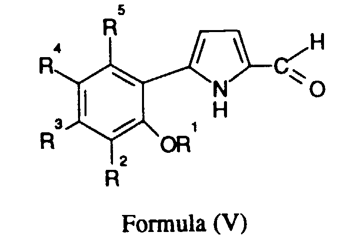 with an amine of formula (III);
and optionally thereafter forming a salt of formula (I).

11. The use of a compound of formula (I) or a physiologically acceptable salt thereof in the manufacture of a medicament for the treatment of a condition which requires modulation of dopamine D₃ receptors.

12. A pharmaceutical composition comprising a compound of formula (I) or a physiologically acceptable salt thereof and a physiologically acceptable carrier.

## Patentansprüche

1. Verbindung der Formel (I): 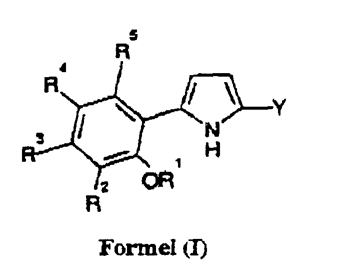 in der
R¹ für einen C₁₋₄-Alkylrest steht; und
R², R³, R⁴ und R⁵ jeweils unabhängig voneinander für ein Wasserstoffatom, Halogen-atom, C₁₋₄-Alkylrest, C₁₋₄-Alkoxyrest, C₁₋₄-Alkoxy-C₁₋₄-alkylrest, C₁₋₄-Alkylsulphonylrest, Trifluormethylsulphonylgruppe; gegebenenfalls substituierten Arylsulphonylrest, gegebenenfalls substituierten Heteroarylsulphonylrest, gegebenenfalls substituierten Aralkylsulphonylrest, gegebenenfalls substituierten Heteroalkarylsulphonylrest, Nitrogruppe, Cyanogruppe, Aminogruppe, Mono- oder Dialkylaminorest, Trifluormethylgruppe, Trifluormethoxygruppe, Hydroxylgruppe, Hydroxyalkylrest, C₁₋₄-Alkylthiorest, C₁₋₄-Alkanoylrest, C₁₋₄-Alkoxycarbonylrest oder SO₂NR⁶R⁷,
wobei R⁶ und R⁷ jeweils unabhängig voneinander für ein Wasserstoffatom, C₁₋₄-Alkylrest oder C₁₋₄-Alkoxy-C₁₋₄-alkylrest stehen oder NR⁶R⁷ einen 3- bis 8- gliedrigen, vollständig gesättigten heterocyclischen Ring, einen 5- bis 8-gliedrigen, teilweise gesättigten heterocyclischen Ring oder einen 5- bis 8-gliedrigen, voliständig gesättigten heterocyclischen Ring, der außer dem Stickstoffatom ein Sauerstoff- oder Schwefelatom enthält, bildet,
stehen; oder
R¹ und R² zusammen eine verknüpfende Kette -(CH₂)ₘOₚ bilden;
(wobei m 2 bis 4 ist und p null oder 1 ist) wobei die Kette gegebenenfalls durch 1 oder 2 C₁₋₄-Alkylreste substituiert sein kann; und
Y für einen Rest der Formel steht: 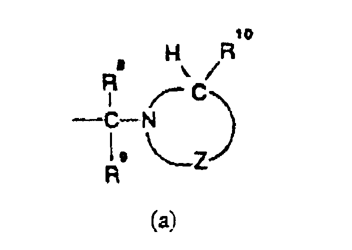 in der
R⁸ und R⁹ unabhängig voneinander für ein Wasserstotfatom, C₁₋₆-Alkylrest, gegebenenfalls substituierten Aryl-C₁₋₆-alkylrest oder gegebenenfalls substituierten Heteroaryl-C₁₋₆-alkylrest stehen;
R¹⁰ für einen gegebenenfalls substituierten Arylrest oder gegebenenfalls substituierten Heteroarylrest steht; und
Z für -(CH₂)ₙ, wobei n 2 bis 8 ist, oder -(CH₂)_{q}CH=CH(CH₂)ᵣ, wobei q und r unabhängig voneinander 1 bis 3 sind, steht;
oder ein Salz davon.

2. Verbindung nach Anspruch 1, in der R¹ für eine Methyl- oder Ethylgruppe steht.

3. Verbindung nach Anspruch 1 oder 2, in der wenigstens einer der Reste R² bis R⁵ ein Wasserstoffatom ist und die weiteren Substituenten gewählt werden aus einem Halogenatom, C₁₋₂-Alkylrest, C₁₋₂-Alkoxyrest, C₁₋₄-Alkylsulphonylrest, Phenylsulphonylgruppe, Benzylsulphonylgruppe und -SO₂NR⁶R⁷, wobei R⁶ und R⁷ für einen C₁₋₄-Alkylrest oder C₁₋₂-Alkoxy-C₁₋₂-alkylrest stehen oder NR⁶R⁷ für einen Ring aus der Gruppe Azetidinyl-, Pyrrolidinyl-, Piperidinyl-, Azacycloheptyl-, 1,2,3,6-Tetrahydropyridinyl-, 4-Morpholinyl- oder 4-Thiomorpholinylgruppe steht.

4. Verbindung nach einem der Ansprüche 1 bis 3, in der R⁸ ein Wasserstoffatom ist und R⁹ ein Wasserstoffatom oder eine Methylgruppe ist.

5. Verbindung nach einem der Ansprüche 1 bis 4, in der Z für -(CH₂)ₙ, wobei n 3, 4 oder 5 ist, oder -(CH₂)_{q}CH=CH(CH₂)ᵣ, wobei die Summe aus q und r 2 oder 3 ist, steht.

6. Verbindung nach einem der Ansprüche 1 bis 5, in der R¹⁰ für eine gegebenenfalls substituierte Phenylgruppe steht.

7. Verbindung nach einem der Ansprüche 1 bis 6, in der der Rest Y für eine 2-Phenylpyrrolidinyl-, 2-Phenylpiperidinyl-, 2-Phenylazacycloheptyl- oder 2-Phenylazacyclooctylgruppe steht.

8. Verbindung nach einem der Ansprüche 1 bis 7, in der das Kohlenstoffatom, das den Rest R¹⁰ trägt, in der R-Konfiguration ist.

9. Verbindung nach Anspruch 1, gewählt aus:
2-(5-Ethylsulphonyl-2-methoxyphenyl)- 5-(1-(2-(R,S)-phenylazacycloheptyl)methyl)-1H-pyrrol, 2-(2-Methoxy-5-N,N-dimethylaminosulphonylphenyl)-5-[1-(2-(R,S)-phenylazacycloheptyl)methyl]-1H-pyrrol, 2-(3-Brom-5-ethylsulphonyl-2-methoxyphenyl)-5-(1-(2-(R,S)-phenylazacycloheptyl)methyl)-1H-pyrrol,2-(5-Ethylsulphonyl-2-methoxyphenyl)-5-(1-(2-(R)-phenylazacycloheptyl)methyl)-1H-pyrrol, 2-(5-Ethylsulphonyl-2-methoxyphenyl)-5-(1-(2-(S)-phenylazacycloheptyl)methyl)-1H-pyrrol, 2-(3-Brom-5-ethylsulphonyl-2-methoxyphenyl)-5-(1-(2-(R)-phenylazacycloheptyl)methyl)-1H-pyrrol, 2-(3-Brom-5-ethylsulphonyl-2-methoxyphenyl)-5-(1-(2-(S)-phenylazacycloheptyl)methyl)-1H-pyrrol, 2-(2-Methoxy-5-dimethylaminosulphonylphenyl)-5-[1-(2-(R,S)-(4-fluorphenyl)azacycloheptyl)methyl]-1H-pyrrol, 2-(3-Brom-5-ethylsulphonyl-2-methoxyphenyl)-5-[1-(2-(R,S)-(4-fluorphenyl)azacycloheptyl)methyl]-1H-pyrrol, 2-(5-Ethylsulphonyl-2-methoxyphenyl)-5-[1-(2-(R,S)-(4-methylphenyl)azacycloheptyl)methyl]-1H-pyrrol, 2-(2-Methoxy-5-(4-morpholinyl)sulphonylphenyl)-5-[1-(2-(R,S)-phenylazacycloheptyl)methyl]-1H-pyrrol, 2-(2-Methoxy-5-(4-morpholinyl)sulphonylphenyl)-5-(1-(2-(R)-phenylazacycloheptyl)methyl)-1H-pyrrol, 2-(2-Methoxy-5-(4-morpholinyl)sulphonylphenyl)-5-(1-(2-(S)-phenylazacycloheptyl)methyl)-1H-pyrrol, 2-[5-(Azetidinylsulphonyl)-2-methoxyphenyl]-5-[1-(2-(R,S)-phenylazacycloheptyl)methyl]-1H-pyrrol, und 2-(5-Isopropylsulphonyl-2-methoxyphenyl)-5-[1-(2-(R,S)-phenylazacycloheptyl)methyl]1H-pyrrol, oder einem Salz davon.

10. Verfahren zur Herstellung einer Verbindung der Formel (I), wie sie in einem der Ansprüche 1 bis 9 definiert ist, wobei das Verfahren umfaßt:
(a) Durchführen einer Mannich-Reaktion mit einer Verbindung der Formel (II): 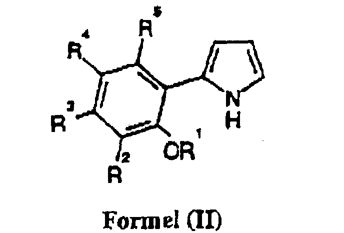 (in der R¹ bis R⁵ wie vorstehend definiert sind)
und einem Amin der Formel (III): 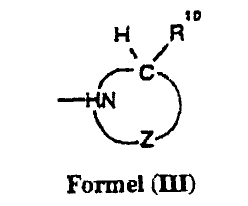 in dem R¹⁰ und Z wie vorstehend definiert sind;
(b) Durchführen einer Vilsmeier-Reaktion mit einer Verbindung der Formel (II) und einem Amid der Formel (IV): 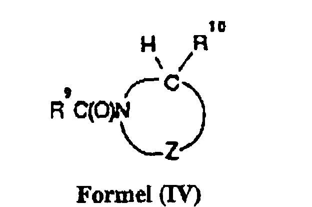 in dem R⁹, R¹⁰ und Z wie vorstehend definiert sind;
und Reduzieren des Zwischenprodukts mit beispielsweise Natriumborhydrid oder -cyanborhydrid;
(c) reduktive Aminierung einer Verbindung der Formel (V) 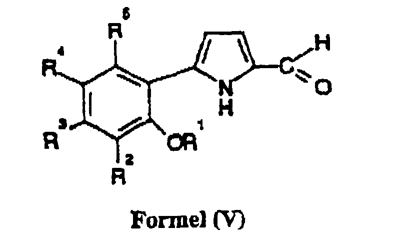 mit einem Amin der Formel (III), um eine Verbindung der Formel (I) herzustellen, in der R⁸ und R⁹ beide für ein Wasserstoffatom stehen;
und gegebenenfalls daraufhin Erzeugen eines Salzes der Formel (I).

11. Verwendung einer Verbindung der Formel (I) oder eines physiologisch verträglichen Salzes davon bei der Herstellung eines Medikaments zur Behandlung einer Erkrankung, die die Regulierung des Dopamin-D₃-Rezeptors erfordert.

12. Arzneimittel, umfassend eine Verbindung der Formel (I) oder ein physiologisch verträgliches Salz davon sowie einen physiologisch verträglichen Träger.

## Revendications

1. Composé de formule (I) : 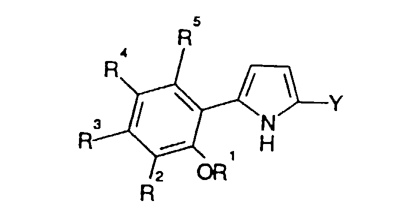 dans laquelle
R¹ représente un groupe alkyle en C₁ à C₄ ; et
R², R³, R⁴ et R⁵ représentent chacun indépendamment l'hydro gène, un halogène, un groupe alkyle en C₁ à C₄, alkoxy en C₁ à C₄, (alkoxy en C₁ à C₄)-(alkyle en C₁ à C₄), alkylsulfonyle en C₁ à C₄, trifluorométhylsulfonyle ; arylsulfonyle facultativement substitué, hétéroarylsulfonyle facultativement substitué, aralkylsulfonyle facultativement substitué, hétéroaralkylsulfonyle facultativement substitué, nitro, cyano, amino, mono- ou dialkylamino, trifluorométhyle, trifluorométhoxy, hydroxyle, hydroxyalkyle, alkylthio en C₁ à C₄, alcanoyle en C₁ à C₄, (alkoxy en C₁ à C₄)carbonyle ou -SO₂NR⁶R⁷ dans lequel R⁶ et R⁷ représentent chacun indépendamment l'hydrogène, un groupe alkyle en C₁ à C₄ ou (alkoxy en C₁ à C₄)-(alkyle en C₁ à C₄), ou bien NR⁶R⁷ forme un noyau hétérocyclique tri- à octogonal totalement saturé, un noyau hétérocyclique penta- à octogonal partiellement saturé ou un noyau hétérocyclique penta- à octogonal totalement saturé qui contient en plus de l'atome d'azote, un atome d'oxygène ou de soufre ; ou
R¹ et R² forment conjointement une chaîne de liaison -(CH₂)ₘOₚ ;
(dans laquelle m a une valeur de 2 à 4 et p est égal à zéro ou 1), chaîne qui peut être facultativement substituée avec un ou deux groupes alkyle en C₁ à C₄ ; et
Y représente un groupe de formule : 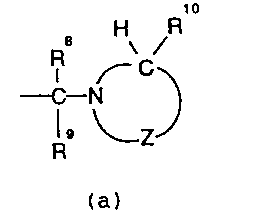 dans laquelle
R⁸ et R⁹ représentent indépendamment l'hydrogène, un groupe alkyle en C₁ à C₆, aryl(alkyle en C₁ à C₆) facultativement substitué ou hétéroaryle (alkyle en C₁ à C₆) facultativement substitué ;
R¹⁰ représente un groupe aryle facultativement substitué ou un groupe hétéroaryle facultativement substitué ; et
Z représente un groupe -(CH₂)ₙ dans lequel n a une valeur de 2 à 8 ou un groupe -(CH₂)_{q}CH=CH(CH₂)ᵣ dans lequel q et r ont indépendamment une valeur de 1 à 3 ;
ou un de ses sels.

2. Composé suivant la revendication 1, dans lequel R¹ représente un groupe méthyle ou éthyle.

3. Composé suivant la revendication 1 ou la revendication 2, dans lequel au moins un des groupes R² à R⁵ représente l'hydrogène et les autres substituants sont choisis entre des substituants halogéno, alkyle en C₁ ou C₂, alkoxy en C₁ ou C₂, alkylsulfonyle en C₁ à C₄, phénylsulfonyle, benzylsulfonyle et -SO₂NR⁶R⁷ dans lesquels R⁶ et R⁷ représentent un groupe alkyle en C₁ à C₄ ou (alkoxy en C₁ ou C₂)-(alkyle en C₁ ou C₂), ou bien NR⁶R⁷ représente un noyau choisi entre les noyaux azétidinyle, pyrrolidinyle, pipéridinyle, azacycloheptyle, 1,2,3,6-tétrahydropyridinyle, 4-morpholinyle et 4-thiomorpholinyle.

4. Composé suivant l'une quelconque des revendications 1 à 3, dans lequel R⁸ représente l'hydrogène et R⁹ représente l'hydrogène ou un groupe méthyle.

5. Composé suivant l'une quelconque des revendications 1 à 4, dans lequel Z représente un groupe -(CH₂)ₙ dans lequel n est égal à 3, 4 ou 5, ou un groupe -(CH₂)_{q}CH=CH(CH₂)ᵣ dans lequel la somme de q et r est égale à 2 ou 3.

6. Composé suivant l'une quelconque des revendications 1 à 5, dans lequel R¹⁰ représente un groupe phényle facultativement substitué.

7. Composé suivant l'une quelconque des revendications 1 à 6, dans lequel le groupe Y représente un groupe 2-phénylpyrrolidinyle, 2-phénylpipéridinyle, 2-phénylazacycloheptyle ou 2-phénylazacyclooctyle.

8. Composé suivant l'une quelconque des revendications 1 à 7, dans lequel l'atome de carbone portant R¹⁰ a la configuration R.

9. Composé suivant la revendication 1, choisi entre les suivants :
2-(5-éthylsulphonyl-2-méthoxyphényl)-5-(1-(2-(R,S)-phénylazacycloheptyl)-méthyl)-1H-pyrrole,
2-(2-méthoxy-5-N,N-diméthylaminosulphonylphényl)-5-[1-(2-(R,S)-phenylazacycloheptyl)méthyl]-1H-pyrrole,
2-(3-bromo-5-éthylsulphonyl-2-méthoxyphényl)-5-(1-(2-(R,S)-phénylazacycloheptyl)méthyl)-1H-pyrrole,
2-(5-éthylsulphonyl-2-méthoxyphényl)-5-(1-(2-(R)-phénylazacycloheptyl)méthyl)1H-pyrrole,
2-(5-éthylsulphonyl-2-méthoxyphényl)-5-(1-(2-(S)-phénylazacycloheptyl)méthyl)-1H-pyrrole,
2-(3-bromo-5-éthylsulphonyl-2-méthoxyphényl)-5-(1-(2-(R)-phénylazacycloheptyl)méthyl)-1H-pyrrole,
2-(3-bromo-5-éthylsulphonyl-2-méthoxyphényl)-5-(1-(2-(S)-phénylazacycloheptyl)-méthyl)-1H-pyrrole,
2-(2-méthoxy-5-diméthylaminosulphonylphényl)-5-[1-(2-(R,S)-(4-fluorophényl)azacycloheptyl)méthyl]-1H-pyrrole,
2-(3-bromo-5-éthylsulphonyl-2-méthoxyphényl)-5-[1-(2-(R,S)-(4-fluorophényl)azacycloheptyl)méthyl]-1H-pyrrole,
2-(5-éthylsulphonyl-2-méthoxyphényl)-5-[1-(2-(R,S)-(4-méthylphényl)azacycloheptyl)méthyl]-1H-pyrrole),
2-(2-méthoxy-5-(4-morpholinyl)sulphonylphényl)-5-[1-(2-(R,S)-phénylazacycloheptyl)méthyl]-1H-pyrrole,
2-(2-méthoxy-5-(4-morpholinyl)sulphonylphényl)-5-(1-(2-(R)-phénylazacycloheptyl)méthyl)-1H-pyrrole,
2-(2-méthoxy-5-(4-morpholinyl)suphonylphényl)-5-(1-(2-(S)-phénylazacycloheptyl)méthyl)-1H-pyrrole,
2-[5-(azétidinylsulphonyl)-2-méthoxyphenyl]-5-[1-(2-(R,S)-phénylazacycloheptyl)méthyl]-1H-pyrrole, et
2-(5-isopropylsulphonyl-2-méthoxyphenyl)-5-[1-(2-(R,S)-phénylazacycloheptyl)méthyl]1H-pyrrole,
ou un de ses sels.

10. Procédé pour la préparation d'un composé de formule (I) répondant à la définition suivant l'une quelconque des revendications 1 à 9, procédé qui comprend :
(a) la conduite d'une réaction de Mannich avec un composé de formule (II) : 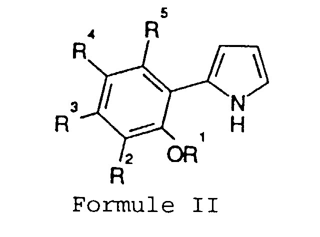 (dans laquelle R¹-R⁵ répondent aux définitions précitées), et une amine de formule (III) : 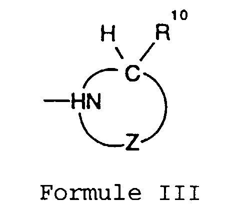 dans laquelle R¹⁰ et Z répondent aux définitions précitées ;
(b) la conduite d'une réaction de Vilsmeier avec un composé de formule (II) et un amide de formule (IV) : 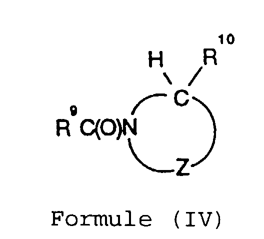 dans laquelle R⁹, R¹⁰ et Z répondent aux définitions précitées ;
et la réduction du produit intermédiaire avec, par exemple, le borohydrure de sodium ou le cyanoborohydrure de sodium ;
c) pour la préparation d'un composé de formule (I) dans laquelle R⁸ et R⁹ représentent l'un et l'autre l'hydrogène, l'amination réductrice d'un composé de formule (V) : 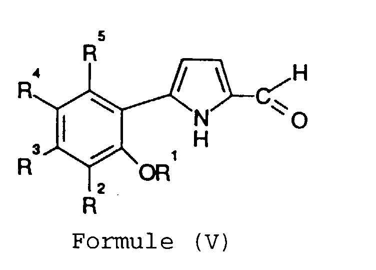 avec une amine de formule (III) ;
et ensuite, facultativement, la formation d'un sel de formule (I).

11. Utilisation de composé de formule (I) ou d'un de ses sels physiologiquement acceptables dans la production de médicaments pour le traitement d'un état qui nécessite une modulation des récepteurs de dopamine D₃.

12. Composition pharmaceutique comprenant un composé de formule (I) ou un de ses sels physiologiquement acceptables et un support physiologiquement acceptable.
